# NEW EUROPEAN PATENT SPECIFICATION

(11) **EP 1 689 869 B2**
(45) Date of publication and mention of the opposition decision: **14.08.2013**
(45) Mention of the grant of the patent: 05.01.2011
(21) Application number: 04799676.4
(22) Date of filing: 05.11.2004
(51) Int. Cl.: C12N 15/75, C12R 1/25, C12P 21/02

(54) **RECOMBINANT MICROORGANISM**
REKOMBINANTER MIKROORGANISMUS
MICRO-ORGANISME RECOMBINE

(30) Priority: 07.11.2003 JP 2003379167
(43) Date of publication of application: 16.08.2006
(60) Divisional application: 10158504.0
(73) Proprietor: KAO CORPORATION, Tokyo 103-8210 (JP)
(72) Inventor: TOHATA, Masatoshi, Kao Corporation Research Lab., Haga-gun, Tochigi 321-3426 (JP); SAWADA, Kazuhisa, Kao Corporation Research Lab., Haga-gun, Tochigi 321-3426 (JP); OZAKI, Katsuya, Kao Corporation Research Lab., Haga-gun, Tochigi 321-3426 (JP); KOBAYASHI, Kazuo, Nara Inatitute of Science&Techn., Ikoma-shi, Nara 630-0101 (JP); OGASAWARA, Naotake, Nara Inatitute of Science&Tech, Ikoma-shi,Nara 630-0101 (JP)
(74) Representative: Vossius & Partner
(86) International application number: PCT/JP2004/016891
(87) International publication number: WO 2005/045013

(56) References cited:
- WO-A-03/083125
- DE-A1- 19 951 765
- WARNER JESSICA B ET AL: "CcpA-independent regulation of expression of the Mg2+-citrate transporter gene citM by arginine metabolism in Bacillus subtilis." JOURNAL OF BACTERIOLOGY, vol. 185, no. 3, February 2003 (2003-02), pages 854-859, XP002346326 ISSN: 0021-9193
- ALI NAIMA OULD ET AL: "Regulation of the acetoin catabolic pathway is controlled by sigma L in Bacillus subtilis" JOURNAL OF BACTERIOLOGY, vol. 183, no. 8, April 2001 (2001-04), pages 2497-2504, XP002346327 ISSN: 0021-9193
- WACKER INGRID ET AL: "The regulatory link between carbon and nitrogen metabolism in Bacillus subtilis: Regulation of the gltAB operon by the catabolite control protein CcpA." MICROBIOLOGY (READING), vol. 149, no. 10, October 2003 (2003-10), pages 3001-3009, XP002346328 ISSN: 1350-0872
- LANGBEIN INES ET AL: "Specific interaction of the RNA-binding domain of the Bacillus subtilis transcriptional antiterminator GlcT with its RNA target, RAT" JOURNAL OF MOLECULAR BIOLOGY, vol. 293, no. 4, 5 November 1999 (1999-11-05), pages 795-805, XP002346329 ISSN: 0022-2836
- EGETER O ET AL: "CATABOLITE REPRESSION MEDIATED BY THE CATABOLITE CONTROL PROTEIN CCPA IN STAPHYLOCOCCUS XYLOSUS" MOLECULAR MICROBIOLOGY, BLACKWELL SCIENTIFIC, OXFORD, GB, vol. 21, no. 4, 1996, pages 739-749, XP002916663 ISSN: 0950-382X
- LEBOEUF CELINE ET AL: "Characterization of the ccpA gene of Enterococcus faecalis: Identification of starvation-inducible proteins regulated by CcpA" JOURNAL OF BACTERIOLOGY, vol. 182, no. 20, October 2000 (2000-10), pages 5799-5806, XP002346330 ISSN: 0021-9193
- DATABASE Geneseq [Online] 29 January 2001 (2001-01-29), "Bacillus sp. KSM-S237 heat resistant alkaline cellulase DNA." XP002346334 retrieved from EBI accession no. GSN:AAA97493 Database accession no. AAA97493
- DATABASE EMBL [Online] 21 May 1993 (1993-05-21), "Bacillus sp. KSM-64 endo-1,4-beta-glucanase gene, complete cds; and unknown gene." XP002346335 retrieved from EBI accession no. EM_PRO:BSENDOGLU Database accession no. M84963
- "Bacterial Nomenclature Up-To-Date; Genus: Bacillus" DSMZ 12 May 2008 (2008-05-12), Retrieved from the Internet: URL:http://www.dsmz.de/microorganisms/bact erial_nomenclature_info.php?genus=Bacillus &show_genus_info=1> [retrieved on 2010-02-26]

## Description

### Technical Field

The present invention relates to a recombinant microorganism which may be used to produce useful proteins or polypeptides, as well as to such proteins and polypeptides.

### Technical Background

Microorganisms are widely used for industrially producing a broad range of useful substances, including alcoholic beverages, certain types of foods such as *miso* and *shoyu,* amino acids, organic acids, nucleic-acid-related substances, antibiotics, sugars, lipids, and proteins. These substances also find diversified uses, including foods, pharmaceuticals, detergents, products for daily use such as cosmetics, and a variety of chemical raw materials.

In industrial production of useful substances by use of microorganisms, improvement of productivity is one major topic of interest, and one approach therefor is breeding of microorganisms through mutagenesis or other genetic means. Recently, in particular, with advancement of microbial genetics and biotechnology, more efficient breeding of useful microorganisms is performed through gene recombination techniques, and in association therewith, host microorganisms for obtaining recombinant genes are under development. For example, *Bacillus subtilis* Marburg No. 168, which has already been confirmed to be safe and have excellent characteristics as a host microorganism, has been further improved.

However, microorganisms inherently possess diversified genes so that they can cope with environmental changes in the natural world, and thus, they do not necessarily exhibit high production efficiency of proteins or similar substances in industrial production, where only limited production media are employed.

### Disclosure of the Invention

The present invention provides a recombinant microorganism prepared by transferring, to a mutant strain of *Bacillus subtilis* from which any of genes *yopO, yvaN, yurK, yozA, yvdE, ykvE, yacP,* and *ylbo,* have been deleted or knocked out, a gene encoding a heterologous protein or polypeptide.

Furthermore, the present invention provides a recombinant microorganism prepared by transferring, to a mutant strain of *Bacillus subtilis* from which any of the genes comA, treR, yvbA, yttP, licR, mntR, yaaT, yyaA, yycH, hprK, yhdK and rsix have been deleted or knocked out, a gene encoding a heterologous protein or polypeptide, wherein a secretion signal region derived from a cellulase gene of a bacterium belonging to the genus *Bacillus,* a transcription initiation regulatory region and a translation initiation regulatory region that are each derived from a 0.6 to 1 kb region upstream of the cellulase gene are ligated to an upstream region of the gene encoding a heterologous protein or polypeptide.

### Brief Description of the Drawings

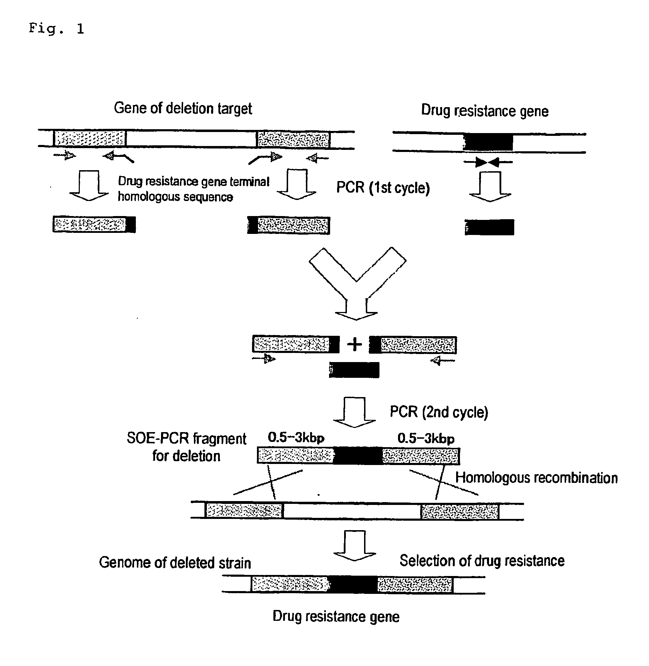

Fig. 1 schematically shows a method for preparing a DNA fragment for deleting a gene through SOE-PCR (SOE: splicing by overlap extension) (see Gene, 77, 61 (1989), and a method for deleting a target gene (replacing the target gene with a drug resistance gene) through use of the DNA.

### Modes for Carrying out the Invention

The present invention is directed to a recombinant microorganism obtained by transferring, into a host microorganism capable of producing protein or polypeptide with increased productivity, a gene encoding a protein or polypeptide, and to a method for producing a protein or polypeptide by use of the recombinant microorganism.

The present inventors have conducted extensive studies on, among many different genes encoded on the genome of a microorganism, genes which are not needed in or which are detrimental to the production of useful proteins or polypeptides, and have found that, when a gene encoding a target protein or polypeptide is transferred to the microorganism *Bacillus subtilis* after a specific gene is deleted or knocked out from the genome of the microorganism, productivity of the target protein or polypeptide is enhanced as compared with the case before the deletion or knocking out.

In the microorganism of the present invention, since genes which are unnecessary in or detrimental to the production or a target protein or polypeptide are deleted or knocked out, waste of culture media, including energy loss, production of byproducts, and reduced specific production rate, is significantly reduced, and in addition, protein and polypeptide can be produced over a prolonged period, whereby a target product can be produced with high efficiency.

In the present invention, homology between amino acid sequences and that between nucleic acid sequences are both determined by use of the Lipman-Pearson method (Science, 227, 1435 (1985)). Specifically, calculation is performed by use of a homology analysis program (Search Homology) developed by genetic information processing software, Genetyx-Win (Software Development Co., Ltd.), with ktup (the unit size to be compared) being set 2.

The parent microorganism for constructing the microorganism of the present invention is *Bacillus subtilis* having genes or genes functionally equivalent thereto as shown in Table 1, wherein the gene may be of wild-type or a mutant. *Bacillus subtilis* is used, from the viewpoint that complete genomic information of this microorganism has already been obtained, and thus genetic engineering techniques and genomic engineering techniques have been established, and that the microorganism has ability to secrete the produced protein extracellularly.

Examples of the target protein or polypeptide to be produced by use of the microorganism of the present invention include enzymes, physiologically active substances, and other proteins and polypeptides which find utility in foods, pharmaceuticals, cosmetics, detergents, fiber-treating agents, clinical assay agents, etc.

Taking *Bacillus subtilis,* which is known to have 4,106 genes on the genome, as an example, one or more genes which are to be deleted or knocked out are any of the *Bacillus subtilis* genes shown in Table 1, or are selected from among the genes functionally equivalent thereto. The present inventors have found that such genes do not directly participate in production of the target protein or polypeptide and are unnecessary for the growth of microorganism in ordinary industrial production media.

The names, numbers, and functions of respective genes in the Tables contained herein conform with the *Bacillus subtilis* genome data reported in Nature, 390, 249-256 (1997) and made public by JAFAN (Japan Functional Analysis Network for *Bacillus subtilis*; BSORF DB) on the Internet (http://bacillus.genome.ad.jp/, renewed June 17, 2003).

**Table 1**

| Name of the gene | Gene ID | Functions or other information of the gene |
|---|---|---|
| *comA* | BG10381 | two-component response regulator |
| *yopO* | BG13648 | deduced transcriptional regulator, spβ prophage protein |
| *treR* | BG11011 | trehalose operon transcriptional repressor (GntR family) |
| *yvbA* | BG14078 | deduced transcriptional regulator (ArsR family) |
| *cspB* | BG10824 | cold shock-related major factor |
| *yvaN* | BG14069 | deduced transcriptional regulator |
| *yttP* | BG13927 | deduced transcriptional regulator (TetR family) |
| *yurK* | BG13997 | deduced transcriptional regulator (GntR family) |
| *yozA* | BG13748 | deduced transcriptional regulator (ArsR family) |
| *licR* | BG11346 | transcriptional regulator (antiterminator), lichenan operon (*licBCAH*) regulation |
| *sigL* | BG10748 | RNA polymerase σ factor (o54) |
| *mntR* | BG11702 | manganese transport regulator |
| *glcT* | BG12593 | transcriptional regulator essential to expression of *ptsGHI* operon (BglG family, antiterminator) |
| *yvdE* | BG12414 | deduced transcriptional regulator (LacI family) |
| *ykvE* | BG13310 | deduced transcriptional regulator (MarR family) |
| *slr* | BG11858 | transcriptional activator for competence- or sporulation-related genes |
| *rocR* | BG10723 | transcriptional activator for arginine-assimilating operon (NtrC family) |
| *ccpA* | BG10376 | carbon source catabolism reppression-related transcriptional regulator (Lacl family) |
| *yaaT* | BG10096 | type-II signal peptidase-like protein |
| *yyaA* | BG10057 | DNA-binding protein SpoOJ-like protein |
| *yycH* | BG11462 | Function unknown (homologous gene has been found in other organisms) |
| *yacP* | BG10158 | Function unknown (homologous gene has been found in other organisms) |
| *hprK* | BG14125 | Hpr protein Ser residue phosphoenzyme/dephosphoenzyme |
| *rsiX* | BG10537 | anti σX factor |
| *yh dK* | BG13017 | Function unknown, related to repression of σM factor expression |
| *ylbO* | BG13367 | expression regulator for gene in σE-related metrocytein |

Genes derived from other microorganisms, preferably from bacteria belonging to the genus *Bacillus,* which have the same functions as any of the *Bacillus subtilis* genes shown in Table 1, or have 70% or more homology with the nucleotide sequence of any of the genes shown in Table 1, preferably 80% or more homology, more preferably 90% or more, further preferably 95% or more, yet more preferably 98% or more, should be interpreted to be functionally equivalent to the genes shown in Table 1, and thus to constitute the genes which are to be deleted or knocked out according to the present invention. In this connection, homology of nucleotides is computed by use of the Lipman-Pearson method (Science, 227, 1435, 1985).

Many of the genes shown in Table 1 which encode *Bacillus subtilis* are regulatory genes participating in activation or suppression of expression of a variety of genes, or genes deduced to be such regulatory genes. The present invention has been attained on the basis of this finding; i.e., the presence of regulatory genes unnecessary in or detrimental to production of protein or polypeptide has now been unveiled in the present invention.

Notably, attention is drawn to the fact that many of the listed "unnecessary" or "detrimental" genes are regulatory genes participating in sugar intake or metabolism, as exemplified by the *glcT* gene, which acts as an antiterminator for a glucose PTS intake operon; the *licT* gene, which acts as an anti-terminator for a lichenan hydrolysis operon; the *treR* gene, which acts as a repressor of trehalose intake and metabolism; and the *hprK* gene and *ccpA* gene, which relate to glucose catabolite repression.

Also, in addition to the regulatory genes involved in sugar intake and metabolism, the *rocR* gene participating in activation of arginine assimilation, and competence-related *comA* gene and *slr* gene, which are also regulatory genes, may be deleted or knocked out, to thereby improve productivity of protein or polypeptide.

The genes shown in Table 1 include the *yhdK* gene, and the *rsiX* gene encoding the anti-EC F sigma factor which suppresses expression of an ECF sigma factor, sigma x. The *yhdK* gene has been reported to participate in suppression of sigma M (Mol. Microbiol., 32, 41, 1999). The *sigL* gene, which encodes sigma L, is also included in the genes of Table 1. This suggests that expression of a gene under regulation by sigma X or sigma M is favorable for production of protein, and conversely, some gene expression under regulation by sigma L is unfavorable.

By deleting or knocking out one or more genes selected from the above-mentioned genes, expression which is unnecessary in or harmful to the production of protein or polypeptide can be prevented, leading to enhanced productivity in such production of protein or polypeptide.

The number of gene(s) to be deleted or knocked out is one or more, preferably two or more, more preferably three or more, even more preferably 5 or more. When a microorganism of the present invention is constructed, deletion or inactivation of a gene or genes other than those mentioned above is possible. In such a case, a more improved effect is expected. An alternative method for achieving the present invention is inactivation, or knocking out, of a target gene by inserting thereto a DNA fragment of another origin or introducing a mutation to the transcription/translation-initiation region of the gene. Preferably, however, the target genes are physically deleted.

In an example procedure for deleting or knocking out the genes, any of the target genes shown in Table 1 is deleted or knocked out according to a plan which has been set up in advance. Alternatively, randomized deletion of genes or mutation by way of knocking out is performed, followed by evaluation on protein productivity and gene analysis.

The target gene may be deleted or knocked out through homologous recombination. That is, a DNA fragment containing a portion of the target gene is cloned with an appropriate plasmid vector to thereby obtain a ring-shaped recombinant plasmid, and the resultant plasmid is transferred into cells of a parent microorganism. Thereafter, through homologous recombination effected in a partial region of the target gene, the target gene on the genome of the parent microorganism is cleaved, thereby completing inactivation of the target gene. Alternatively, the target gene is knocked out by substitution or insertion of a base, or a linear DNA fragment containing a region outside the target gene sequence but not containing the target gene may be constituted through PCR or a similar method, and the thus-engineered gene or fragment is transferred into a cell of a parent microorganism. At two sites outside the mutation within the target gene in the genome of the parent microorganism genome, or at two regions outside the target gene sequence, double crossing-over homologous recombination is caused to occur, to thereby attain substitution with a gene fragment in which the target gene on the genome is deleted or knocked out.

Particularly when the parent microorganism used to construct the microorganism of the present invention is *Bacillus subtilis,* since several reports have already described methods for deleting or knocking out the target gene (see, for example, Mol. Gen. Genet., 223, 268 1990), repetition of any of such methods may be followed, to thereby produce a host microorganism of the present invention.

Randomized gene deletion or inactivation may be performed through use of a method similar to the above-described method for inducing homologous recombination by use of a randomly cloned DNA fragment, or by way of irradiation of a parent microorganism with gamma rays or similar rays.

Next will be described in more detail a deletion method employing double crossing over by use of a DNA fragment designed for the deletion purpose, the DNA fragment being prepared through SOE-PCR (Gene, 77, 61, 1989). However, in the present invention, the method for deleting genes is not limited to only the below-described method.

The DNA fragment use for the deletion purpose is a fragment constructed such that a drug resistant marker gene is inserted between a ca. 0.5 to 3 kb upstream sequence which flanks and is upstream of the gene to be deleted, and a ca. 0.5 to 3 kb downstream sequence which flanks and is downstream of the same gene. In the first cycle of PCR, the following three fragments are prepared: the upstream and the downstream fragments, which are to be deleted, and the drug resistant marker gene. The primers to be used in this step may, for example, be those specifically designed so that an upstream 10-30 base pair sequence of a drug resistance gene is added to the lower end of the upstream fragment, and a downstream 10-30 base pair sequence of the drug resistance marker gene is added to the upper end of the downstream fragment (Fig. 1).

Next, using three PCR fragments prepared in the first cycle as templates, the second cycle of PCR is performed by use of an upper primer of the upstream fragment and a lower primer of the downstream fragment. This step causes annealing with the drug resistance marker gene fragment in the sequence of the above-engineered drug resistance marker gene, and through PCR amplification, there can be obtained a DNA fragment with the drug resistance marker gene inserted between the upstream fragment and the downstream fragment (Fig. 1).

When a chloramphenicol-resistant gene is employed as a drug resistance marker gene, a DNA fragment for deleting a gene can be obtained through SOE-PCR under typical conditions described in literature (see, for example, PCR Protocols. Current Methods and Applications, Edited by B. A. White, Humana Press, pp. 251 (1993), Gene, 77, 61, 1989), by use of a primer set such as that shown in Table 2 and a conventional enzyme kit for PCR (e.g., Pyrobest DNA Polymerase (product of Takara Shuzo)).

When the thus-obtained DNA fragment for effecting gene deletion is introduced into cells through the competent method or a similar method, intracellular genetic recombination occurs in homologous regions which are present upstream and downstream of the gene to be deleted. Thus, cells in which the target gene has been substituted by a drug resistance gene can be selectively separated through employment of a drug resistance marker (Fig. 1). Specifically, when a DNA fragment for gene deletion prepared by use of a primer set shown in Table 2 is introduced into cells, colonies which have grown on an agar culture medium containing chloramphenicol are separated, and deletion of the target gene by way of substitution by the chloramphenicol-resistant gene is confirmed through an appropriate method such as PCR employing a genome as a template.

Subsequently, when a gene encoding a target protein or polypeptide is transferred to a host mutant microorganism strain from which any of the *Bacillus subtilis* genes shown in Table 1, or one or more genes selected from among the genes corresponding thereto has been deleted or knocked out, the microorganism of the present invention can be obtained.

No particular limitation is imposed on the gene encoding the target protein or polypeptide. Examples of the protein and polypeptide include physiologically-active peptides and enzymes for industrial purposes such as detergents, foods, fibers, feeds, chemicals, medicine, and diagnostic agents. Industrial enzymes may be functionally grouped into oxidoreductases, transferases, hydrolases, lyases, isomerases, and ligases/synthetases. Preferably, hydrolases such as cellulase, α-amylase, and protease may be used. Specific examples include cellulase belonging to family 5 in the classification of hydrolase (Bioche M. J., 280, 309, 1991); in particular, cellulase derived from a microorganism, more particularly cellulase derived from the genus *Bacillus.* Other specific examples of the types of industrial enzymes include alkaline cellulase which is derived from the genus *Bacillus* and has an amino-acid of SEQ ID NOs: 2 or 4, and cellulase which has an another amino-acid sequence having 70% homology with said amino-acid sequence, preferably 80% homology, more preferably 90%, further preferably 95%, still further preferably 98% or more.

Specific examples of α-amylase include α-amylase derived from a microorganism, preferably liquefied amylase derived from the genus *Bacillus.* More specific examples include alkaline amylase which is derived from the genus *Bacillus* and has an amino-acid sequence of SEQ ID NO: 6, and amylase which has another amino-acid sequence having 70% homology with said amino-acid sequence, preferably 80% homology, more preferably 90%, further preferably 95%, particularly preferably 98% or more. The homology of the amino-acid sequence is calculated by the Lipman-Pearson method (Science, 227, 1435 (1985)). Specific examples of protease include serine protease and metallo-protease which are derived from microorganisms, particularly those belonging to the genus *Bacillus.*

Preferably, a gene coding for a target protein or polypeptide has, on its upstream region thereof, one or more regulatory regions relating to transcription, translation, or secretion of the gene (specially, one or more regions selected from among a transcription initiation regulatory region including a promoter and a transcription initiation site; a translation initiation region including a ribosome-binding site and a start codon; and a secretion signal peptide region) properly ligated thereto. Preferably, it is preferred that three regions consisting of the transcription initiation regulatory region, the translation initiation regulatory region, and the secretion signal region be ligated to the target gene. Further preferably, the secretion signal peptide region is one that originates from the cellulase gene of a microorganism belonging to the genus *Bacillus,* and the transcription initiation region and the translation initiation region is a 0.6 to 1 kb region upstream of the cellulase gene. In one preferred example, a transcription initiation regulatory region, a translation initiation region, and a secretion signal peptide region of a cellulase gene derived from a microorganism belonging to the genus *Bacillus* disclosed in, for example, Japanese Patent Application Laid-Open (*kokai*) Nos. 2000-210081 and 190793/1990; i.e., a cellulase gene derived from KSM-S237 strain (FERM BP-7875) or KSM-64 strain (FERM BP-2886), is properly ligated to a structural gene of the target protein or polypeptide. More specifically, preferred DNA fragments to be ligated include a nucleotide sequence of base numbers 1 to 659 of SEQ ID NO: 1; a nucleotide sequence of base numbers 1 to 696 of a cellulase gene of SEQ ID NO: 3; a DNA fragment having a nucleotide sequence having 70% homology with any one of said nucleotide sequences, preferably 80% homology, more preferably 90%, further preferably 95%, even more preferably 98% or more; or a DNA fragment having a nucleotide sequence lacking a portion of any one of said nucleotide sequences. Preferably, one of these DNA fragments is properly ligated to a structural gene of the target protein or polypeptide. As used herein, a DNA fragment having a nucleotide sequence lacking a portion of any one of the above-mentioned nucleotide sequences is intended to mean a DNA fragment which has functions relating to transcription, translation, and secretion of the gene, without having a portion of any one of the above-mentioned nucleotide sequences.

The recombinant microorganism of the present invention can be obtained by a conventional transformation technique in which a recombinant plasmid containing a DNA fragment which includes a gene encoding the target protein or polypeptide, and is ligated to a proper plasmid vector is transferred into a host microorganism cell. Alternatively, the recombinant microorganism may be obtained making use of a DNA fragment prepared by ligating the above DNA fragment to a proper region which is homologous with a certain portion of the host microorganism genome, and inserted directly into a host microorganism genome.

The target protein or polypeptide obtained by use of the recombinant microorganism of the present invention may be produced in such a manner that a corresponding cell strain is inoculated onto a culture medium containing assimilable carbon sources and nitrogen sources, and other essential components; the cell strain is cultured through a conventional microorganism culturing method; and subsequently, protein or polypeptide is collected and purified.

Through the aforementioned procedure, a host mutant microorganism strain in which any of the *Bacillus subtilis* genes shown in Table 1 can be engineered. In addition, by use of such a mutant strain, a recombinant microorganism can be produced. Thus, a useful protein or polypeptide can be effectively produced through employment of the mutant strain or the recombinant microorganism.

The method for constructing a recombinant microorganism according to the present invention, and the method for producing cellulase and α-amylase by use of the recombinant microorganism will next be described in detail, centering on working examples for constructing recombinant strain belonging to *Bacillus subtilis* from which the *ccpA* gene (BG10376) of *Bacillus subtilis* has been deleted.

### Examples

### Example 1

A genome DNA sample, serving as a template, extracted from *Bacillus subtilis* 168 strain and two primer sets (ccpA-AF and ccpA-A/CmR; and ccpA-B/CmF and ccpA-BR) shown in Table 2 were used to prepare a 0.6 kb fragment (A) flanking the upstream side of the *ccpA* gene on the genome and a 0.6 kb fragment (B) flanking the downstream side of the *ccpA* gene. A chloramphenicol-resistant gene of plasmid pC194 (J. Bacteriol. 150 (2), 815 (1982))) was inserted into the XbaI-BamHI cleavages site of plasmid pUC18, to thereby prepare a recombinant plasmid pCBB 31. The recombinant plasmid pCBB and a primer set consisting of CmF and CmR shown in Table 2 were used to prepare a 1 kb fragment (C) containing the chloramphenicol-resistant gene. Subsequently, SOE-PCR was performed by use of the primers ccpA-AF and ccpA-BR shown in Table 2, and by use of the thus-prepared three fragments (A), (B), and (C) in combination as templates, a 2.2 kb DNA fragment in which the fragments (A), (B), and (C) were ligated in this sequence was prepared (see Fig. 1). By use of the thus-prepared DNA fragment, *Bacillus subtilis* 168 strain was transformed through the competent method. Colonies grown in an LB agar medium containing chloramphenicol were collected as transformants. The genome of the above-obtained-transformant was extracted, and PCR performed thereon confirmed that the ccpA gene had been deleted and substituted by a chloramphenicol-resistant gene.

**Table 2-1**

| Primer | Nucleotide sequence | SEQ ID NO: |
|---|---|---|
| comA-AF | AAGGATGATAATCCGTCCCGTG | 7 |
| comA-A/CmR | GTTATCCGCTCACAATTCGGATGGTCATCAATCACTAG | 8 |
| comA-B/CmF | CGTCGTGACTGGGAAAACTGCGAAATCAGACGGTGTAC | 9 |
| comA-BR | CGTCGCCTATCGGCGGGCAC | 10 |
| yop0-AF | ATGTATATAGGAGGTTGGTGGTATG | 11 |
| yop0-A/CmR | GTTATCCGCTCACAATTCGCTCTCACATGTCAACCTCC | 12 |
| yop0-B/CmF | CGTCGTGACTGGGAAAACAGATGAGAAAGGAGGAGAAG | 13 |
| yop0-BR | ATAACTGTTACTATATAATGGCC | 14 |
| treR-AF | GCTGGGGATGACGAATCCGA | 15 |
| treR-A/CmR | GTTATCCGCTCACAATTCTCACCTTCATTATGGACCAC | 16 |
| treR-B/CmF | CGTCGTGACTGGGAAAACCACGGTCTCGACAAATTCCG | 17 |
| treR-BR | GTTGCCAAGCGCGATATAGG | 18 |
| yvbA-AF | TATACAGGGATTATCAGTATTGAGC | 19 |
| yvbA-A/CmR | GTTATCCGCTCACAATTCTTTTCTCCTTGTTGGATCTG | 20 |
| yvbA-B/CmF | CGTCGTGACTGGGAAAACGGGGATAACGATTTATGAAG | 21 |
| yvbA-BR | TTTTGTAATAATGATATGAAGCTAGTGTTG | 22 |
| cspB-AF | ATATCCAGCCCTGCCTCTTC | 23 |
| cspB-A/CmR | | 24 |
| cspB-B/CmF | | 25 |
| cspB-BR | TCCTGTTTGGGCTCCTGTTG | 26 |
| yvaN-AF | TGTTTATGTATGGCGGCCTGCGGGAC | 27 |
| yvaN-A/CmR | GTTATCCGCTCACAATTCAGCTTTCCATATATCTCACC | 28 |
| yvaN-B/CmF | CGTCGTGACTGGGAAAACACGGTCTGCTGATGACTGAC | 29 |
| yvaN-BR | GCGTTTACTTAAGATGTCGA | 30 |
| yttP-AF | TTTCTAGCGTTTCGGCAAATTGAGTTAAG | 31 |
| yttP-A/CmR | GTTATCCGCTCACAATTCCTTACTTTCATACGGCTCAC | 32 |
| yttP-B/CmF | CGTCGTGACTGGGAAAACGAGACGTGGCGCTCACCAAC | 33 |
| yttP-BR | CGGATTAAAAAAAGAATATCGCGGACAGC | 34 |
| yurK-AF | TGCCGCTGCCCGCCGGAGAG | 35 |

**Table 2-2**

| | | |
|---|---|---|
| yurK-A/CmR | GTTATCCGCTCACAATTCAAGGTGTAGAACTTCCGTTG | 36 |
| yurlC-B/CmF | CGTCGTGACTGGGAAAACACCATCAACAGCCCCTACAC | 37 |
| yurK-BR | TCAAATAAAGGCGGCATTCAGTCC | 38 |
| yozA-AF | ATAATGGTATCCAAATCCACGC | 39 |
| yozA-A/CmR | GTTATCCGCTCACAATTCATTCAGTCATATGTATCACC | 40 |
| yozA-B/CmF | CGTCGTGACTGGGAAAACGATCCATCATACACAGCATG | 41 |
| yozA-BR | CACTTCTCAACGGAGGGGATTTCACATC | 42 |
| licR-AF | TAATGGAGGAGAGAAGGCCG | 43 |
| licR-A/CmR | GTTATCCGCTCACAATTCAGTCGCCCATGAAGCATGAG | 44 |
| licR-B/CmF | CGTCGTGACTGGGAAAACACCAAAAAATGCTGAGCTGACAGC | 45 |
| licR-BR | TTGCCAATGATGAGGAAAAAGGAACC | 46 |
| sigL-AF | CTGAACGTCTTGAATAAAAAAGCAGG | 47 |
| sigL-A/CmR | GTTATCCGCTCACAATTCGCTGAAGTTTCATATCCATC | 48 |
| sigL-B/CmF | CGTCGTGACTGGGAAAACATTCCGTCATCGGCAGCGAG | 49 |
| sigL-BR | AGCGGTTTACAAGTTGGAGG | 50 |
| mntR-AF | ATTTCAGAAGGCATACTTCAAG | 51 |
| mntR-A/CmR | GTTATCCGCTCACAATTCCATACTTGGTGTTGTCATCG | 52 |
| mntR-B/CmF | CGTCGTGACTGGGAAAACCATAATCAGTAAAAAGGCGGTC | 53 |
| mntR-BR | TTCTGACCGCTCTGGCAACC | 54 |
| glcT-AF | ATAATGCCCGCTTCCCAACC | 55 |
| glcT-A/CmR | GTTATCCGCTCACAATTCCGATCCTCAGCTCCTTTGTC | 56 |
| glcT-B/CmF | CGTCGTGACTGGGAAAACTCATCTGATACCGATTAACC | 57 |
| glcT-BR | CAACTGAATCCGAAGGAATG | 58 |
| yvdE-AF | TCGGGGTCATGCCGAGCGGT | 59 |
| yvdE-A/CmR | GTTATCCGCTCACAATTCCAATGTTGCCATTTTCATCC | 60 |
| yvdE-B/CmF | CGTCGTGACTGGGAAAACTTGTACGAGAATCAACGCTG | 61 |
| yvdE-BR | CACGGCAATGCATTCTTCGG | 62 |
| ykvE-AF | AGATCTGTCGGCCAGGTTTAC | 63 |
| ykvE-A/CmR | GTTATCCGCTCACAATTCTGATTTTTCTGTCATGTCTC | 64 |
| ykvE-B/CmF | CGTCGTGACTGGGAAAACGGTAGAGATGTGCACCGAAA | 65 |
| ykvE-BR | GAGTCAGACGGCATCGATGA | 66 |
| slr-AF | TTCTGATTCATTTTCACTGCTGG | 67 |
| slr-A/CmR | GTTATCCGCTCACAATTCAACGGATAATTCTTCCAATC | 68 |
| slr-B/CmF | CGTCGTGACTGGGAAAACTGTCCATGAAGTCAAATCC | 69 |
| slr-BR | CGCTGAAATATTCTCTCGCA | 70 |
| rocR-AF | CGCCGCTTTCACCGCGGATTC | 71 |
| rocR-A/CmR | GTTATCCGCTCACAATTCCTTTGACCACTGTATGAACC | 72 |

**Table 2-3**

| | | |
|---|---|---|
| rocR-B/CmF | CGTCGTGACTGGGAAAACACTCGTCTAACGAATAATCC | 73 |
| rocR-BR | TGTCATCACGGAATTTGACG | 74 |
| ccpA-AF | CCAAATTATCCTTTGTGAGCGCGGAATCAG | 75 |
| ccpA-A/CmR | GTTATCCGCTCACAATTCCGTAGATCGTAATATTGCTC | 76 |
| ccpA-B/CmF | CGTCGTGACTGGGAAAACAGCTTAGAAAGTCAACCAAG | 77 |
| ccpA-BR | TTTGAGCATCAGCACAAGCC | 78 |
| yaaT-AF | TGTAGCAGAAGCAGTCGAATT | 79 |
| yaaT-A/Cm2R | CTAATGGGTGCTTTAGTTGACAATTACGCAGCTGTCATGT | 80 |
| yaaT-B/Cm2F | CTGCCCCGTTAGTTGAAGAACTGATAAACCGTGAAAAAGTG | 81 |
| yaaT-RV | CCTTTGAAAAAGGCTCCCGT | 82 |
| yyaA-AF | GTTTTCCAAGTCTGCCGATAAAAATATGC | 83 |
| yyaA-A/CmR | GTTATCCGCTCACAATTCATGCTTCATGTACCTACACC | 84 |
| yyaA-B/CmF | CGTCGTGACTGGGAAAACCAATTAACGATTCGCATACC | 85 |
| yyaA-BR | AAAAAGAAGAAGTCACAGTACAGAACGTGG | 86 |
| yycH-AF | ATTTTTCGCCATCTTGAATTTTC | 87 |
| yycH-A/Cm2R | CTAATGGGTGCTTTAGTTGGATGATCCTCTCGTTGAACTG | 88 |
| yycH-B/Cm2F | CTGCCCCGTTAGTTGAAGGGATGAGCCTTCAGAAAAGTT | 89 |
| yycH-BR | GCCGGACAGAGATCTGTATG | 90 |
| yacP-B/Cm4F | GAAGAAGGTTTTTATGTTGACGCTTTTTTGCCCAATACTGTATAA | 91 |
| yacP-B/Cm4R | CAAAAAAGCGTCAACATAAAAACCTTCTTCAACTAACGGGGCAGG | 92 |
| yacP-BR | AAGACGAGTACTTTTCTCTCTAAATCACTT | 93 |
| yacP-AF | AACTCGATCAAATGGTGACAGGACAGCATC | 94 |
| yacP-A/Cm4F | GGAGAATAAAGACCCTCTTCAACTAAAGCACCCATTAGTTCAACA | 95 |
| yacP-A/Cm4R | TGCTTTAGTTGAAGAGGGTCTTTATTCTCCCACAGGGTTTCGTTT | 96 |
| hprK-B/Cm4F | TTTTTATATTACAGCGAGTTGGCGTTAAATGAATGAAGCGATAGA | 97 |
| hprK-B/Cm4R | ATTTAACGCCAACTCGCTGTAATATAAAAACCTTCTTCAACTAAC | 98 |
| hprK-BR | TTGATTGATGATAAATTCAGGCAGGTGCAG | 99 |
| hprK-AF | CAAAGCTTGAGAAATGTTCCCATGCTCTTG | 100 |
| hprK-A/Cm4F | CAGGAGGAACATATCTCTTCAACTAAAGCACCCATTAGTTCAACA | 101 |
| hprK-A/Cm4R | TGCTTTAGTTGAAGAGATATGTTCCTCCTGTTCCGGGCTGCCCCG | 102 |
| rsiX-AF | ATTCCAGTTACTCGTAATATAGTTG | 103 |
| rsiX-A/CmR | GTTATCCGCTCACAATTCACTTCATCATCCATTAGCTC | 104 |
| rsiX-B/CmF | CGTCGTGACTGGGAAAACCTGCTCCAAATCCGATTTCC | 105 |
| rsiX-BR | GTCCTGCATTTTTCGAAGTCTGG | 106 |
| yhdK-AF | TACACATCCTTCAAACAAGTCTGAACAAAC | 107 |

**Table 2-4**

| | | |
|---|---|---|
| yhdK-A/Cm4R | TGCTTTAGTTGAAGATTACCAGTTCCATAATTCCACCTCGCCGAC | 108 |
| yhdK-B/Cm4F | TTTTTATATTACAGCGTGTGTATACCATTGTATCTGTAGATACGA | 109 |
| yhdK-BR | GCTATGATCATTGTAACGAAAGGAAAGGGG | 110 |
| yhdK-A/Cm4F | TTATGGAACTGGTAATCTTCAACTAAAGCACCCATTAGTTCAACA | 111 |
| yhdK-B/Cm4R | CAATGGTATACACACGCTGTAATATAAAAACCTTCTTCAACTAAC | 112 |
| ylb0-AF | AATCTGAACAAGAAAAAGGAGCTGCTCCTC | 113 |
| ylb0-A/Cm4R | TGCTTTAGTTGAAGAATTCAATCTCCCTCCATGTCAGCTTATTTA | 114 |
| ylb0-B/Cm4F | TTTTTATATTACAGCAGAAACGCCTGAATGAACCGGCCCTATAG | 115 |
| ylb0-BR | TGTTTGACAAAGGTAGAACGTCTGCTTATC | 116 |
| ylb0-A/Cm4F | GGAGGGAGATTGAATTCTTCAACTAAAGCACCCATTAGTTCAACA | 117 |
| ylb0-B/Cm4R | ATTTCAGGCGTTTCTGCTGTAATATAAAAACGTTCTTCAACTAAC | 118 |
| CmF | GAATTGTGAGCGGATAAC | 119 |
| CmR | GTTTTCCCAGTCACGACG | 120 |
| Cm2F | CAACTAAAGCACCCATTAG | 121 |
| Cm2R | CTTCAACTAACGGGGCAG | 122 |

### Example 2

In a manner similar to that described in Example 1, sporulation gene-deleted strains into which a chloramphenicol-resistant gene had been introduced by way of substitution in place of the below-described deleted genes were separated through use of a DNA fragment for effecting deletion prepared from an adequate primer set selected from among various primer sets shown in Table 2; i.e., gene-AF, gene-A/CmR, gene-B/CmF, gene-BR, CmF, and CmR. The gene deleted from the genome was *comA, yopO, treR, yvbA, yvaN, yttP, yurK, yozA, licR, sigL, mntR, glcT, ykvE, slr, rocR, yyaA,* or *rsiX.*

### Example 3

In a manner similar to that described in Example 2, a DNA fragment for deletion was prepared by use of an adequate primer set selected from among the gene-AF, gene-A/Cm2R, gene-B/Cm2F, gene-BR, Cm2F, and Cm2R, which are shown in Table 2. By use of the thus-prepared DNA fragment, sporulation gene-deleted strains into which a chloramphenicol-resistant gene had been introduced by way of substitution in place of the below-described deleted genes were separated. The gene deleted from the genome was *cspB, yvdE, yaaT, yycH,* or *ylbO.*

### Example 4

In a manner similar to that described in Example 2, a DNA fragment for effecting deletion was prepared from an adequate primer set selected from among the gene-AF, gene-A/Cm4R, gene-B/Cm4F, gene-BR, Cm4F, and Cm4R, which are shown in Table 2. By use of the thus-prepared DNA fragment, sporulation gene-deleted strains into which a chloramphenicol-resistant gene had been introduced by way of substitution in place of deleted genes; *yacP, hprK,* and *yhdK,* were separated.

### Example 5

To each of the gene-deleted strains obtained in Examples 1 to 4 and to *Bacillus subtilis* 168 strain serving as a control, a recombinant plasmid pHY-S237 was introduced through the protoplast transformation method. The recombinant plasmid pHY-S237 was prepared by inserting a DNA fragment (3.1 kb) encoding an alkaline cellulase derived from *Bacillus* sp. KSM-S237 strain (Japanese Patent Application Laid-Open (*kokai*) No. 2000-210081) into the restriction enzyme *Bam*HI cleavage site of a shuttle vector pHY300 PLK. Each of the thus-obtained cell strains was shake-cultured in an LB medium (5 mL) overnight at 30°C. The culture broth (0.03 mL) was inoculated to a 2 × L-maltose medium (2% tryptone, 1% yeast extract, 1% NaCl, 7.5% maltose, 7.5 ppm manganese sulfate 4-5 hydrate, and 15 ppm tetracycline), followed by shake culturing at 30°C for three days. After completion of culturing, cells were removed through centrifugation, and alkaline cellulase activity of the supernatant obtained from the culture was determined, thereby calculating the amount of the alkaline cellulase secreted from the cells during culturing; i.e., the amount of the extracellularly produced alkaline cellulase. As is clear from Table 3, more effective production, or secretion, of alkaline cellulase has been confirmed in the case where a gene-deleted strain was employed as a host, as compared with the control 168 strain (wild type strain).

**Table 3**

| Name of deleted gene | Gene ID | Gene size (bp) | Size of deleted fragment (bp) | Amount of produced (secreted) alkaline cellulase (relative value) |
|---|---|---|---|---|
| *comA* | BG10381 | 645 | 588 | 160 |
| *yopO* | BG13648 | 213 | 169 | 154 |
| *treR* | BG11011 | 717 | 656 | 139 |
| *yvbA* | BG14078 | 273 | 210 | 137 |
| *cspB* | BG10824 | 204 | 171 | 132 |
| *yvaN* | BG14069 | 408 | 379 | 124 |
| *yttP* | BG13927 | 624 | 590 | 121 |
| *yurK* | BG13997 | 729 | 677 | 118 |
| *yozA* | BG13748 | 324 | 289 | 117 |
| *licR* | BG11346 | 1926 | 1889 | 116 |
| *sigL* | BG10748 | 1311 | 1256 | 114 |
| *mntR* | BG11702 | 429 | 399 | 114 |
| *glcT* | BG12593 | 858 | 811 | 110 |
| *yvdE* | BG12414 | 951 | 916 | 109 |
| *ykvE* | BG13310 | 438 | 356 | 108 |
| *slr* | BG11858 | 459 | 394 | 105 |
| *rocR* | BG10723 | 1386 | 1359 | 128 |
| *ccpA* | BG10376 | 1005 | 957 | 205 |
| *yaaT* | BG10096 | 828 | 828 | 127 |
| *yyaA* | BG10057 | 852 | 816 | 113 |
| *yycH* | BG11462 | 1368 | 1368 | 146 |
| *yacP* | BG10158 | 513 | 513 | 156 |
| *hprK* | BG14125 | 933 | 933 | 196 |
| *rsiX* | BG10537 | 1107 | 1068 | 125 |
| *yhdK* | BG13017 | 291 | 228 | 114 |
| *JlbO* | BG13367 | 582 | 582 | 136 |
| None (Wild type) | - | - | - | 100 |

### Example 6

To each of the gene-deleted strains obtained in Examples 1 to 4 and to *Bacillus subtilis* 168 strain serving as a control, recombinant plasmid pHSP-K38 was introduced through the protoplast transformation method. The recombinant plasmid pHSP-K38 was prepared by inserting, into the restriction enzyme BagII-xbaI cleavage site of a shuttle vector pHY300 PLK, a 2.1 kb fragment (SEQ ID No: 5) prepared by ligating an upstream 0.6 kb fragment (SEQ ID NO: 3) including portions of a promoter region and a signal sequence region of an alkaline cellulase gene with an upstream side of a DNA fragment (1.5 kb) encoding a mature enzyme region (Aspl-Gln480) of an alkaline amylase gene derived from *Bacillus* sp. KSM-K38 strain (Japanese Patent Application Laid-Open (*kokai*) No. 2000-1884882, Eur. J. Biochem., 268, 2974 (2001)). Each of the thus-obtained cell strains was shake-cultured in an LB medium (5 mL) overnight at 30°C. The culture broth (0.03 mL) was inoculated to a 2 × L-maltose medium (2% tryptone, 1% yeast extract, 1% NaCl, 7.5% maltose, 7.5 ppm manganese sulfate 4-5 hydrate, and 15 ppm tetracycline), followed by shake culturing at 30°C for three to six days. After completion of culturing, cells were removed through centrifugation, and alkaline amylase activity of the supernatant obtained from the culture was determined, thereby calculating the amount of the alkaline amylase secreted from the cells during culturing; i.e., the amount of the extracellularly produced alkaline amylase. As is clear from Table 3, more effective production, or secretion, of alkaline amylase has been confirmed in the case where a gene-deleted strain was employed as a host, as compared with the control 168 strain (wild type strain).

**Table 4**

| Name of deleted gene | Gene ID | Gene size (bp) | Size of deleted fragment (bp) | Amount of produced (secreted) alkaline amylase (relative value) |
|---|---|---|---|---|
| Cultured for 3 days | | | | |
| *slr* | BG11858 | 459 | 394 | 178 |
| *treR* | BG11011 | 717 | 656 | 124 |
| *yopO* | BG13648 | 213 | 169 | 364 |
| *yvaN* | BG14069 | 408 | 379 | 148 |
| *yvbA* | BG14078 | 273 | 210 | 171 |
| None (Wild type) | - | - | - | 100 |
| Culture for 5 days (Wild type) | | | | |
| *cspB* | BG10824 | 204 | 171 | 195 |
| *rocR* | BG10723 | 1386 | 1359 | 215 |
| *sigL* | BG10748 | 1311 | 1256 | 204 |
| *glcT* | BG12593 | 858 | 811 | 132 |
| *yvdE* | BG12414 | 951 | 916 | 127 |
| *yacP* | BG10158 | 513 | 513 | 110 |
| None (Wild type) | - | - | - | 100 |
| Cultured for 6days | | | | |
| *yycH* | BG11462 | 1368 | 1368 | 120 |
| *licR* | BG11346 | 1926 | 1889 | 122 |
| None (Wild type) | - | - | - | 100 |

## Claims

1. A recombinant microorganism prepared by transferring, to a mutant strain of *Bacillus subtilis* from which any of the genes yopO, yvaN, yurK, yozA, yvdE, ykvE, yacP, and ylbO have been deleted or knocked out, a gene encoding a heterologous protein or polypeptide.

2. The recombinant microorganism as claimed in claim 1, wherein one or more regions selected from among a transcription initiation regulatory region, a translation initiation regulatory region, and a secretion signal region is ligated to an upstream region of a gene encoding a heterologous protein or polypeptide.

3. The recombinant microorganism as claimed in claim 2, wherein the one or more regions are three regions constituted by a transcription initiation regulatory region, a translation initiation regulatory region, and a secretion signal region.

4. The recombinant microorganism as claimed in claim 2 or 3, wherein the secretion signal region is derived from a cellulase gene of a bacterium belonging to the genus *Bacillus* and the transcription initiation regulatory region and the translation initiation regulatory region are each derived from a 0.6 to 1 kb region upstream of the cellulase gene.

5. A recombinant microorganism prepared by transferring, to a mutant strain of *Bacillus subtilis* from which any of the genes comA, treR yvbA, yttP, licR, mntR, yaaT, yyaA, yycH, hprK, yhdK and rsiX have been deleted or knocked out, a gene encoding a heterologous protein or polypeptide, wherein a secretion signal region derived from a cellulase gene of a bacterium belonging to the genus *Bacillus,* a transcription initiation regulatory region and a translation initiation regulatory region that are each derived from a 0.6 to 1 kb regions upstream of the cellulase gene are ligated to an upstream region of the gene encoding a heterologous protein or polypeptide.

6. The recombinant microorganism as claimed in claim 3 or 5, wherein the three regions constituted by the transcription initiation regulatory region, the translation initiation regulatory region, and the secretion signal region are
(a) a nucleotide sequence of base numbers 1 to 659 of a cellulase gene of SEQ ID NO: 1;
(b) a nucleotide sequence of base numbers 1 to 696 of a cellulase gene of SEQ ID NO: 3;
(c) a DNA fragment having a nucleotide sequence having 70% homology with either of the nucleotide sequences of (a) or (b); or
(d) a DNA fragment having a nucleotide sequence lacking a portion of any one of the nucleotide sequences of (a) to (c), but said fragment having functions relating to transcription, translation, and secretion of the genes to be expressed.

7. A method for producing a protein or polypeptide by use of a recombinant microorganism as defined in any one of claims 1 to 6.

## Patentansprüche

1. Ein rekombinanter Mikroorganismus, hergestellt durch Transfer eines Gens, das ein heterologes Protein oder Polypeptid codiert, in eine Mutante von *Bacillus subtilis,* in dem irgendeines der Gene yopO, yvaN, yurK, yozA, yvdE, ykvE, yacP und ylbO deletiert oder inaktiviert wurde.

2. Der rekombinante Mikroorganismus gemäß Patentanspruch 1, wobei eine oder mehrere Regionen, ausgewählt aus der Gruppe bestehend aus einer die Initiation der Transkription regulierenden Region, einer die Initiation der Translation regulierenden Region und einer Sekretionssignalregion, an eine upstream-Region des Gens, welches das heterologe Protein oder Polypeptid codiert, ligiert ist.

3. Der rekombinante Mikroorganismus gemäß Patentanspruch 2, wobei die eine oder die mehreren Regionen drei Regionen sind und sich aus einer die Initiation der Transkription regulierenden Region, einer die Initiation der Translation regulierenden Region und einer Sekretionssignalregion zusammensetzen.

4. Der rekombinante Mikroorganismus gemäß Patentanspruch 2 oder 3, wobei die Sekretionssignalregion von einem Zellulase-Gen eines Bakteriums des Genus *Bacillus* stammt und die die Initiation der Transkription regulierende Region sowie die die Initiation der Translation regulierende Region beide aus einer Region stammen, die 0,6 kb bis 1 kb upstream von besagtem Zellulase-Gen liegt.

5. Ein rekombinanter Mikroorganismus hergestellt durch Transfer eines Gens, das ein heterologes Protein oder Polypeptid codiert, in eine Mutante von *Bacillus subtilis,* in dem irgendeines der Gene comA, treR, yvbA, yttP, licR, mntR, yaaT, yyaA, yycH, hprK, yhdK und rsiX deletiert oder inaktiviert wurde, wobei eine Sekretionssignalregion, die von einem Zellulase-Gen eines Bakteriums des Genus *Bacillus* stammt, eine die Initiation der Transkription regulierende Region und eine die Initiation der Translation regulierende Region, die beide von einer Region stammen, die 0,6 bis 1 kb upstream des Zellulase-Gens liegt, an eine upstream-Region des Gens ligiert sind, die ein heterologes Protein oder Polypeptid codiert.

6. Der rekombinante Mikroorganismus gemäß Patenanspruch 3 oder 5, wobei die drei Regionen, die sich aus der die Initiation der Transkription regulierenden Region, der die Initiation der Translation regulierenden Region und der Sekretionssignalregion zusammensetzen:
(a) eine Nucleotidsequenz gemäß der Basen 1 bis 659 eines Zellulase-Gens gemäß SEQ ID NO:1 sind;
(b) eine Nucleotidsequenz gemäß der Basen 1 bis 696 eines Zellulase-Gens gemäß SEQ ID NO:3 sind;
(c) ein DNA-Fragment mit einer Nucleotidsequenz sind, die 70% Homologie mit einer der Nucleotidsequenzen von (a) oder (b) hat;
(d) ein DNA-Fragment sind, dem ein Teil der Nucleotidsequenz gemäß der Nucleotidsequenzen von (a) bis (c) fehlt, wobei besagtes Fragment funktional in Bezug auf Transkription, Translation und Sekretion des zu exprimierenden Gens ist.

7. Ein Verfahren zur Herstellung eines Proteins oder Polypeptids unter Verwendung eines rekombinanten Mikroorganismus wie in einem der Ansprüche 1 bis 6 definiert.

## Revendications

1. Micro-organisme recombiné préparé par le transfert, vers une souche mutante de *Bacillus subtilis* de laquelle l'un quelconque des gènes yopO, yvaN, yurK, yozA, yvdE, ykvE, yacP et ylbO ont été supprimés ou désactivés, d'un gène codant une protéine hétérologue ou un polypeptide hétérologue.

2. Micro-organisme recombiné selon la revendication 1, dans lequel une ou plusieurs régions choisies parmi une région régulatrice de l'initiation de la transcription, une région régulatrice de l'initiation de la traduction et une région de signal de sécrétion sont ligaturés à une région en amont d'un gène codant une protéine hétérologue ou un polypeptide hétérologue.

3. Micro-organisme recombiné selon la revendication 2, dans lequel la ou les régions sont trois régions constituées par une région régulatrice de l'initiation de la transcription, une région régulatrice de l'initiation de la traduction et une région de signal de sécrétion.

4. Micro-organisme recombiné selon la revendication 2 ou 3, dans lequel la région de signal de sécrétion dérive d'un gène de cellulase d'une bactérie appartenant au genre *Bacillus* et la région régulatrice de l'initiation de la transcription et la région régulatrice de l'initiation de la traduction dérivent chacune d'une région de 0,6 kb à 1 kb se trouvant en amont du gène de cellulase.

5. Micro-organisme recombiné préparé par le transfert, vers une souche mutante de *Bacillus subtilis* de laquelle l'un quelconque des gènes comA, treR, yvbA, yttP, licR, mntR, yaaT, yyaA, yycH, hprK, yhdK et rsiX ont été supprimés ou désactivés, d'un gène codant une protéine hétérologue ou un polypeptide hétérologue, dans lequel une région de signal de sécrétion dérivée d'un gène de cellulase d'une bactérie appartenant au genre *Bacillus,* une région régulatrice de l'initiation de la transcription et une région régulatrice de
l'initiation de la traduction, dont chacune dérive d'une région de 0,6 à 1 kb se trouvant en amont du gène de cellulase, sont ligaturés à une région en amont d'un gène codant une protéine hétérologue ou un polypeptide hétérologue.

6. Micro-organisme recombiné selon les revendications 3 ou 5, dans lequel les trois régions constituées de la région régulatrice de l'initiation de la transcription, la région régulatrice de l'initiation de la traduction et la région de signal de sécrétion sont :
(a) une séquence nucléotidique composée des bases numéro 1 à 659 d'un gène de cellulase de SEQ ID NO:1 ;
(b) une séquence nucléotidique composée des bases numéro 1 à 696 d'un gène de cellulase de SEQ ID NO:3 ;
(c) un fragment d'ADN possédant une séquence nucléotidique présentant une homologie de 70% avec soit la séquence nucléotidique de (a), soit la séquence nucléotidique de (b) ; ou
(d) un fragment d'ADN possédant une séquence nucléotidique dépourvue d'une partie de l'une quelconque des séquences nucléotidiques de (a) à (c), ledit fragment possédant cependant des fonctions relatives à la transcription, la traduction et la sécrétion des gènes à exprimer.

7. Procédé de production d'une protéine ou d'un polypeptide par l'utilisation d'un micro-organisme recombiné défini dans l'une quelconque des revendications 1 à 6.


### SEQUENCE LISTING

<110> KAO CORPORATION
<120> Recombinant microorganisms
<130> KS0795
<150> JP 2003-379167
   <151> 2003. 11.7
<160> 122
<170> PatentIn Ver. 2. 1
<210> 1
   <211> 3150
   <212> DNA
   <213> Bacillus sp. KSM-S237
<220>
   <221> CDS
   <222> (573).. (3044)
   <223>
<220>
   <221> sig_peptide
   <222> (573).. (659)
   <223>
<220>
   <221> mat_peptide
   <222> (660).. ()
   <223>
<400> 1
   gatttgccga tgcaacaggc ttatatttag aggaaatttc tttttaaatt gaatacggaa      60
   taaaatcagg taaacaggtc ctgattttat ttttttgagt tttttagaga actgaagatt     120
   gaaataaaag tagaagacaa aggacataag aaaattgcat tagttttaat tatagaaaac     180
   gcctttttat aattatttat acctagaacg aaaatactgt ttcgaaagcg gtttactata     240
   aaaccttata ttccggctct tttttaaaac agggggtaaa aattcactct agtattctaa     300
   tttcaacatg ctataataaa tttgtaagac gcaatatgca tctctttttt tacgatatat     360
   gtaagcggtt aaccttgtgc tatatgccga tttaggaagg ggggtagatt gagtcaagta     420
   gtaataatat agataactta taagttgttg agaagcagga gagcatctgg gttactcaca     480
   agttttttta aaactttaac gaaagcactt tcggtaatgc ttatgaattt agctatttga     540
   ttcaattact ttaaaaatat ttaggaggta at atg atg tta aga aag aaa aca       593
                                       Met Met Leu Arg Lys Lys Thr
                                                       -25
   aag cag ttg att tct tcc att ctt att tta gtt tta ctt cta tct tta       641
   Lys Gln Leu Ile Ser Ser Ile Leu Ile Leu Val Leu Leu Leu Ser Leu
           -20                 -15                 -10
   ttt ccg gca gct ctt gca gca gaa gga aac act cgt gaa gac aat ttt       689
   Phe Pro Ala Ala Leu Ala Ala Glu Gly Asn Thr Arg Glu Asp Asn Phe
       -5                  -1  1               5                   10
   aaa cat tta tta ggt aat gac aat gtt aaa cgc cct tct gag gct ggc       737
   Lys His Leu Leu Gly Asn Asp Asn Val Lys Arg Pro Ser Glu Ala Gly
                   15                  20                  25
   gca tta caa tta caa gaa gtc gat gga caa atg aca tta gta gat caa       785
   Ala Leu Gln Leu Gln Glu Val Asp Gly Gln Met Thr Leu Val Asp Gln
               30                  35                  40
   cat gga gaa aaa att caa tta cgt gga atg agt aca cac gga tta cag        833
   His Gly Glu Lys Ile Gln Leu Arg Gly Met Ser Thr His Gly Leu Gln
           45                  50                  55
   tgg ttt cct gag atc ttg aat gat aac gca tac aaa gct ctt tct aac      881
   Trp Phe Pro Glu Ile Leu Asn Asp Asn Ala Tyr Lys Ala Leu Ser Asn
       60                  65                  70
   gat tgg gat tcc aat atg att cgt ctt gct atg tat gta ggt gaa aat      929
   Asp Trp Asp Ser Asn Met Ile Arg Leu Ala Met Tyr Val Gly Glu Asn
   75                  80                  85                  90
   ggg tac gct aca aac cct gag tta atc aaa caa aga gtg att gat gga      977
   Gly Tyr Ala Thr Asn Pro Glu Leu Ile Lys Gln Arg Val Ile Asp Gly
                   95                  100                 105
   att gag tta gcg att gaa aat gac atg tat gtt att gtt gac tgg cat      1025
   Ile Glu Leu Ala Ile Glu Asn Asp Met Tyr Val Ile Val Asp Trp His
               110                 115                 120
   gtt cat gcg cca ggt gat cct aga gat cct gtt tat gca ggt gct aaa      1073
   Val His Ala Pro Gly Asp Pro Arg Asp Pro Val Tyr Ala Gly Ala Lys
           125                 130                 135
   gat ttc ttt aga gaa att gca gct tta tac cct aat aat cca cac att      1121
   Asp Phe Phe Arg Glu Ile Ala Ala Leu Tyr Pro Asn Asn Pro His Ile
       140                 145                 150
   att tat gag tta gcg aat gag ccg agt agt aat aat aat ggt gga gca      1169
   Ile Tyr Glu Leu Ala Asn Glu Pro Ser Ser Asn Asn Asn Gly Gly Ala
   155                 160                 165                 170
   ggg att ccg aat aac gaa gaa ggt tgg aaa gcg gta aaa gaa tat gct      1217
   Gly Ile Pro Asn Asn Glu Glu Gly Trp Lys Ala Val Lys Glu Tyr Ala
                   175                 180                 185
   gat cca att gta gaa atg tta cgt aaa agc ggt aat gca gat gac aac      1265
   Asp Pro Ile Val Glu Met Leu Arg Lys Ser Gly Asn Ala Asp Asp Asn
               190                 195                 200
   att atc att gtt ggt agt cca aac tgg agt cag cgt ccg gac tta gca      1313
   Ile Ile Ile Val Gly Ser Pro Asn Trp Ser Gln Arg Pro Asp Leu Ala
           205                 210                 215
   gct gat aat cca att gat gat cac cat aca atg tat act gtt cac ttc      1361
   Ala Asp Asn Pro Ile Asp Asp His His Thr Met Tyr Thr Val His Phe
       220                 225                 230
   tac act ggt tca cat gct gct tca act gaa agc tat ccg tct gaa act      1409
   Tyr Thr Gly Ser His Ala Ala Ser Thr Glu Ser Tyr Pro Ser Glu Thr
   235                 240                 245                 250
   cct aac tct gaa aga gga aac gta atg agt aac act cgt tat gcg tta      1457
   Pro Asn Ser Glu Arg Gly Asn Val Met Ser Asn Thr Arg Tyr Ala Leu
                   255                 260                 265
   gaa aac gga gta gcg gta ttt gca aca gag tgg gga acg agt caa gct      1505
   Glu Asn Gly Val Ala Val Phe Ala Thr Glu Trp Gly Thr Ser Gln Ala
               270                 275                 280
   agt gga gac ggt ggt cct tac ttt gat gaa gca gat gta tgg att gaa      1553
   Ser Gly Asp Gly Gly Pro Tyr Phe Asp Glu Ala Asp Val Trp Ile Glu
           285                 290                 295
   ttt tta aat gaa aac aac att agc tgg gct aac tgg tct tta acg aat      1601
   Phe Leu Asn Glu Asn Asn Ile Ser Trp Ala Asn Trp Ser Leu Thr Asn
       300                 305                 310
   aaa aat gaa gta tct ggt gca ttt aca cca ttc gag tta ggt aag tct      1649
   Lys Asn Glu Val Ser Gly Ala Phe Thr Pro Phe Glu Leu Gly Lys Ser
   315                 320                 325                 330
   aac gca acc aat ctt gac cca ggt cca gat cat gtg tgg gca cca gaa      1697
   Asn Ala Thr Asn Leu Asp Pro Gly Pro Asp His Val Trp Ala Pro Glu
                   335                 340                 345
   gaa tta agt ctt tct gga gaa tat gta cgt gct cgt att aaa ggt gtg      1745
   Glu Leu Ser Leu Ser Gly Glu Tyr Val Arg Ala Arg Ile Lys Gly Val
               350                 355                 360
   aac tat gag cca atc gac cgt aca aaa tac acg aaa gta ctt tgg gac      1793
   Asn Tyr Glu Pro Ile Asp Arg Thr Lys Tyr Thr Lys Val Leu Trp Asp
           365                 370                 375
   ttt aat gat gga acg aag caa gga ttt gga gtg aat tcg gat tct cca      1841
   Phe Asn Asp Gly Thr Lys Gln Gly Phe Gly Val Asn Ser Asp Ser Pro
       380                 385                 390
   aat aaa gaa ctt att gca gtt gat aat gaa aac aac act ttg aaa gtt      1889
   Asn Lys Glu Leu Ile Ala Val Asp Asn Glu Asn Asn Thr Leu Lys Val
   395                 400                 405                 410
   tcg gga tta gat gta agt aac gat gtt tca gat ggc aac ttc tgg gct      1937
   Ser Gly Leu Asp Val Ser Asn Asp Val Ser Asp Gly Asn Phe Trp Ala
                   415                 420                 425
   aat gct cgt ctt tct gcc aac ggt tgg gga aaa agt gtt gat att tta      1985
   Asn Ala Arg Leu Ser Ala Asn Gly Trp Gly Lys Ser Val Asp Ile Leu
               430                 435                 440
   ggt gct gag aag ctt aca atg gat gtt att gtt gat gaa cca acg acg      2033
   Gly Ala Glu Lys Leu Thr Met Asp Val Ile Val Asp Glu Pro Thr Thr
           445                 450                 455
   gta gct att gcg gcg att cca caa agt agt aaa agt gga tgg gca aat      2081
   Val Ala Ile Ala Ala Ile Pro Gln Ser Ser Lys Ser Gly Trp Ala Asn
       460                 465                 470
   cca gag cgt gct gtt cga gtg aac gcg gaa gat ttt gtc cag caa acg      2129
   Pro Glu Arg Ala Val Arg Val Asn Ala Glu Asp Phe Val Gln Gln Thr
   475                 480                 485                 490
   gac ggt aag tat aaa gct gga tta aca att aca gga gaa gat gct cct      2177
   Asp Gly Lys Tyr Lys Ala Gly Leu Thr Ile Thr Gly Glu Asp Ala Pro
                   495                 500                 505
   aac cta aaa aat atc gct ttt cat gaa gaa gat aac aat atg aac aac      2225
   Asn Leu Lys Asn Ile Ala Phe His Glu Glu Asp Asn Asn Met Asn Asn
               510                 515                 520
   atc att ctg ttc gtg gga act gat gca gct gac gtt att tac tta gat      2273
   Ile Ile Leu Phe Val Gly Thr Asp Ala Ala Asp Val Ile Tyr Leu Asp
           525                 530                 535
   aac att aaa gta att gga aca gaa gtt gaa att cca gtt gtt cat gat      2321
   Asn Ile Lys Val Ile Gly Thr Glu Val Glu Ile Pro Val Val His Asp
       540                 545                 550
   cca aaa gga gaa gct gtt ctt cct tct gtt ttt gaa gac ggt aca cgt      2369
   Pro Lys Gly Glu Ala Val Leu Pro Ser Val Phe Glu Asp Gly Thr Arg
   555                 560                 565                 570
   caa ggt tgg gac tgg gct gga gag tct ggt gtg aaa aca gct tta aca      2417
   Gln Gly Trp Asp Trp Ala Gly Glu Ser Gly Val Lys Thr Ala Leu Thr
                   575                 580                 585
   att gaa gaa gca aac ggt tct aac gcg tta tca tgg gaa ttt gga tat      2465
   Ile Glu Glu Ala Asn Gly Ser Asn Ala Leu Ser Trp Glu Phe Gly Tyr
               590                 595                 600
   cca gaa gta aaa cct agt gat aac tgg gca aca gct cca cgt tta gat      2513
   Pro Glu Val Lys Pro Ser Asp Asn Trp Ala Thr Ala Pro Arg Leu Asp
           605                 610                 615
   ttc tgg aaa tct gac ttg gtt cgc ggt gag aat gat tat gta gct ttt      2561
   Phe Trp Lys Ser Asp Leu Val Arg Gly Glu Asn Asp Tyr Val Ala Phe
   620 625 630
   gat ttc tat cta gat cca gtt cgt gca aca gaa ggc gca atg aat atc 2609
   Asp Phe Tyr Leu Asp Pro Val Arg Ala Thr Glu Gly Ala Met Asn Ile
   635 640 645 650
   aat tta gta ttc cag cca cct act aac ggg tat tgg gta caa gca cca 2657
   Asn Leu Val Phe Gln Pro Pro Thr Asn Gly Tyr Trp Val Gln Ala Pro
   655 660 665
   aaa acg tat acg att aac ttt gat gaa tta gag gaa gcg aat caa gta 2705
   Lys Thr Tyr Thr Ile Asn Phe Asp Glu Leu Glu Glu Ala Asn Gln Val
   670 675 680
   aat ggt tta tat cac tat gaa gtg aaa att aac gta aga gat att aca 2753
   Asn Gly Leu Tyr His Tyr Glu Val Lys Ile Asn Val Arg Asp Ile Thr
   685 690 695
   aac att caa gat gac acg tta cta cgt aac atg atg atc att ttt gca 2801
   Asn Ile Gln Asp Asp Thr Leu Leu Arg Asn Met Met Ile Ile Phe Ala
   700 705 710
   gat gta gaa agt gac ttt gca ggg aga gtc ttt gta gat aat gtt cgt 2849
   Asp Val Glu Ser Asp Phe Ala Gly Arg Val Phe Val Asp Asn Val Arg
   715 720 725 730
   ttt gag ggg gct gct act act gag ccg gtt gaa cca gag cca gtt gat 2897
   Phe Glu Gly Ala Ala Thr Thr Glu Pro Val Glu Pro Glu Pro Val Asp
   735 740 745
   cct ggc gaa gag acg cca cct gtc gat gag aag gaa gcg aaa aaa gaa 2945
   Pro Gly Glu Glu Thr Pro Pro Val Asp Glu Lys Glu Ala Lys Lys Glu
   750 755 760
   caa aaa gaa gca gag aaa gaa gag aaa gaa gca gta aaa gaa gaa aag 2993
   Gln Lys Glu Ala Glu Lys Glu Glu Lys Glu Ala Val Lys Glu Glu Lys
   765 770 775
   aaa gaa gct aaa gaa gaa aag aaa gca gtc aaa aat gag gct aag aaa 3041
   Lys Glu Ala Lys Glu Glu Lys Lys Ala Val Lys Asn Glu Ala Lys Lys
   780 785 790
   aaa taatctatta aactagttat agggttatct aaaggtctga tgtagatctt 3094
   Lys
   795
   ttagataacc tttttcttgc ataactggac acagagttgt tattaaagaa agtaag 3150
<210> 2
   <211> 824
   <212> PRT
   <213> Bacillus sp. KSM-S237
<400> 2
   Met Met Leu Arg Lys Lys Thr Lys Gln Leu Ile Ser Ser Ile Leu Ile
   -25 -20 -15
   Leu Val Leu Leu Leu Ser Leu Phe Pro Ala Ala Leu Ala Ala Glu Gly
   -10 -5 -1 1
   Asn Thr Arg Glu Asp Asn Phe Lys His Leu Leu Gly Asn Asp Asn Val
   5 10 15
   Lys Arg Pro Ser Glu Ala Gly Ala Leu Gln Leu Gln Glu Val Asp Gly
   20 25 30 35
   Gln Met Thr Leu Val Asp Gln His Gly Glu Lys Ile Gln Leu Arg Gly
   40 45 50
   Met Ser Thr His Gly Leu Gln Trp Phe Pro Glu Ile Leu Asn Asp Asn
   55 60 65
   Ala Tyr Lys Ala Leu Ser Asn Asp Trp Asp Ser Asn Met Ile Arg Leu
   70 75 80
   Ala Met Tyr Val Gly Glu Asn Gly Tyr Ala Thr Asn Pro Glu Leu Ile
   85 90 95
   Lys Gln Arg Val Ile Asp Gly Ile Glu Leu Ala Ile Glu Asn Asp Met
   100 105 110 115
   Tyr Val Ile Val Asp Trp His Val His Ala Pro Gly Asp Pro Arg Asp
   120 125 130
   Pro Val Tyr Ala Gly Ala Lys Asp Phe Phe Arg Glu Ile Ala Ala Leu
   135 140 145
   Tyr Pro Asn Asn Pro His Ile Ile Tyr Glu Leu Ala Asn Glu Pro Ser
   150 155 160
   Ser Asn Asn Asn Gly Gly Ala Gly Ile Pro Asn Asn Glu Glu Gly Trp
   165 170 175
   Lys Ala Val Lys Glu Tyr Ala Asp Pro Ile Val Glu Met Leu Arg Lys
   180 185 190 195
   Ser Gly Asn Ala Asp Asp Asn Ile Ile Ile Val Gly Ser Pro Asn Trp
   200 205 210
   Ser Gln Arg Pro Asp Leu Ala Ala Asp Asn Pro Ile Asp Asp His His
   215 220 225
   Thr Met Tyr Thr Val His Phe Tyr Thr Gly Ser His Ala Ala Ser Thr
   230 235 240
   Glu Ser Tyr Pro Ser Glu Thr Pro Asn Ser Glu Arg Gly Asn Val Met
   245 250 255
   Ser Asn Thr Arg Tyr Ala Leu Glu Asn Gly Val Ala Val Phe Ala Thr
   260 265 270 275
   Glu Trp Gly Thr Ser Gln Ala Ser Gly Asp Gly Gly Pro Tyr Phe Asp
   280 285 290
   Glu Ala Asp Val Trp Ile Glu Phe Leu Asn Glu Asn Asn Ile Ser Trp
   295 300 305
   Ala Asn Trp Ser Leu Thr Asn Lys Asn Glu Val Ser Gly Ala Phe Thr
   310 315 320
   Pro Phe Glu Leu Gly Lys Ser Asn Ala Thr Asn Leu Asp Pro Gly Pro
   325 330 335
   Asp His Val Trp Ala Pro Glu Glu Leu Ser Leu Ser Gly Glu Tyr Val
   340 345 350 355
   Arg Ala Arg Ile Lys Gly Val Asn Tyr Glu Pro Ile Asp Arg Thr Lys
   360 365 370
   Tyr Thr Lys Val Leu Trp Asp Phe Asn Asp Gly Thr Lys Gln Gly Phe
   375 380 385
   Gly Val Asn Ser Asp Ser Pro Asn Lys Glu Leu Ile Ala Val Asp Asn
   390 395 400
   Glu Asn Asn Thr Leu Lys Val Ser Gly Leu Asp Val Ser Asn Asp Val
   405 410 415
   Ser Asp Gly Asn Phe Trp Ala Asn Ala Arg Leu Ser Ala Asn Gly Trp
   420 425 430 435
   Gly Lys Ser Val Asp Ile Leu Gly Ala Glu Lys Leu Thr Met Asp Val
   440 445 450
   Ile Val Asp Glu Pro Thr Thr Val Ala Ile Ala Ala Ile Pro Gln Ser
   455 460 465
   Ser Lys Ser Gly Trp Ala Asn Pro Glu Arg Ala Val Arg Val Asn Ala
   470 475 480
   Glu Asp Phe Val Gln Gln Thr Asp Gly Lys Tyr Lys Ala Gly Leu Thr
   485 490 495
   Ile Thr Gly Glu Asp Ala Pro Asn Leu Lys Asn Ile Ala Phe His Glu
   500 505 510 515
   Glu Asp Asn Asn Met Asn Asn Ile Ile Leu Phe Val Gly Thr Asp Ala
   520 525 530
   Ala Asp Val Ile Tyr Leu Asp Asn Ile Lys Val Ile Gly Thr Glu Val
   535 540 545
   Glu Ile Pro Val Val His Asp Pro Lys Gly Glu Ala Val Leu Pro Ser
   550 555 560
   Val Phe Glu Asp Gly Thr Arg Gln Gly Trp Asp Trp Ala Gly Glu Ser
   565 570 575
   Gly Val Lys Thr Ala Leu Thr Ile Glu Glu Ala Asn Gly Ser Asn Ala
   580 585 590 595
   Leu Ser Trp Glu Phe Gly Tyr Pro Glu Val Lys Pro Ser Asp Asn Trp
   600 605 610
   Ala Thr Ala Pro Arg Leu Asp Phe Trp Lys Ser Asp Leu Val Arg Gly
   615 620 625
   Glu Asn Asp Tyr Val Ala Phe Asp Phe Tyr Leu Asp Pro Val Arg Ala
   630 635 640
   Thr Glu Gly Ala Met Asn Ile Asn Leu Val Phe Gln Pro Pro Thr Asn
   645 650 655
   Gly Tyr Trp Val Gln Ala Pro Lys Thr Tyr Thr Ile Asn Phe Asp Glu
   660 665 670 675
   Leu Glu Glu Ala Asn Gln Val Asn Gly Leu Tyr His Tyr Glu Val Lys
   680 685 690
   Ile Asn Val Arg Asp Ile Thr Asn Ile Gln Asp Asp Thr Leu Leu Arg
   695 700 705
   Asn Met Met Ile Ile Phe Ala Asp Val Glu Ser Asp Phe Ala Gly Arg
   710 715 720
   Val Phe Val Asp Asn Val Arg Phe Glu Gly Ala Ala Thr Thr Glu Pro
   725 730 735
   Val Glu Pro Glu Pro Val Asp Pro Gly Glu Glu Thr Pro Pro Val Asp
   740 745 750 755
   Glu Lys Glu Ala Lys Lys Glu Gln Lys Glu Ala Glu Lys Glu Glu Lys
   760 765 770
   Glu Ala Val Lys Glu Glu Lys Lys Glu Ala Lys Glu Glu Lys Lys Ala
   775 780 785
   Val Lys Asn Glu Ala Lys Lys Lys
   790 795
<210> 3
   <211> 3332
   <212> DNA
   <213> Bacillus sp. KSM-64
<220>
   <221> CDS
   <222> (610).. (3075)
   <223>
<220>
   <221> sig_peptide
   <222> (610).. (696)
   <223>
<220>
   <221> wat_peptide
   <222> (697).. ()
   <223>
<400> 3
   agtacttacc attttagagt caaaagatag aagccaagca ggatttgccg atgcaaccgg 60
   cttatattta gagggaattt ctttttaaat tgaatacgga ataaaatcag gtaaacaggt 120
   cctgatttta tttttttgaa tttttttgag aactaaagat tgaaatagaa gtagaagaca 180
   acggacataa gaaaattgta ttagttttaa ttatagaaaa cgcttttcta taattattta     240
   tacctagaac gaaaatactg tttcgaaagc ggtttactat aaaaccttat attccggctc     300
   tttttttaaa cagggggtga aaattcactc tagtattcta atttcaacat gctataataa    360
   atttgtaaga cgcaatatac atcttttttt tatgatattt gtaagcggtt aaccttgtgc     420
   tatatgccga tttaggaagg gggtagattg agtcaagtag tcataattta gataacttat     480
   aagttgttga gaagcaggag agaatctggg ttactcacaa gttttttaaa acattatcga     540
   aagcactttc ggttatgctt atgaatttag ctatttgatt caattacttt aataatttta     600
   ggaggtaat atg atg tta aga aag aaa aca aag cag ttg att tct tcc att     651
             Met Met Leu Arg Lys Lys Thr Lys Gln Leu Ile Ser Ser Ile
                             -25                 -20
   ctt att tta gtt tta ctt cta tct tta ttt ccg aca gct ctt gca gca       699
   Leu Ile Leu Val Leu Leu Leu Ser Leu Phe Pro Thr Ala Leu Ala Ala
   -15                 -10                 -5              -1   1
   gaa gga aac act cgt gaa gac aat ttt aaa cat tta tta ggt aat gac       747
   Glu Gly Asn Thr Arg Glu Asp Asn Phe Lys His Leu Leu Gly Asn Asp
               5                   10                  15
   aat gtt aaa cgc cct tct gag gct ggc gca tta caa tta caa gaa gtc       795
   Asn Val Lys Arg Pro Ser Glu Ala Gly Ala Leu Gln Leu Gln Glu Val
           20                  25                  30
   gat gga caa atg aca tta gta gat caa cat gga gaa aaa att caa tta       843
   Asp Gly Gln Met Thr Leu Val Asp Gln His Gly Glu Lys Ile Gln Leu
       35                  40                  45
   cgt gga atg agt aca cac gga tta caa tgg ttt cct gag atc ttg aat       891
   Arg Gly Met Ser Thr His Gly Leu Gln Trp Phe Pro Glu Ile Leu Asn
   50                  55                  60                  65
   gat aac gca tac aaa gct ctt gct aac gat tgg gaa tca aat atg att       939
   Asp Asn Ala Tyr Lys Ala Leu Ala Asn Asp Trp Glu Ser Asn Met Ile
                   70                  75                  80
   cgt cta gct atg tat gtc ggt gaa aat ggc tat gct tca aat cca gag       987
   Arg Leu Ala Met Tyr Val Gly Glu Asn Gly Tyr Ala Ser Asn Pro Glu
               85                  90                  95
   tta att aaa agc aga gtc att aaa gga ata gat ctt gct att gaa aat       1035
   Leu Ile Lys Ser Arg Val Ile Lys Gly Ile Asp Leu Ala Ile Glu Asn
           100                 105                 110
   gac atg tat gtc atc gtt gat tgg cat gta cat gca cct ggt gat cct       1083
   Asp Met Tyr Val Ile Val Asp Trp His Val His Ala Pro Gly Asp Pro
       115                 120                 125
   aga gat ccc gtt tac gct gga gca gaa gat ttc ttt aga gat att gca       1131
   Arg Asp Pro Val Tyr Ala Gly Ala Glu Asp Phe Phe Arg Asp Ile Ala
   130                 135                 140                 145
   gca tta tat cct aac aat cca cac att att tat gag tta gcg aat gag       1179
   Ala Leu Tyr Pro Asn Asn Pro His Ile Ile Tyr Glu Leu Ala Asn Glu
                   150                 155                 160
   cca agt agt aac aat aat ggt gga gct ggg att cca aat aat gaa gaa       1227
   Pro Ser Ser Asn Asn Asn Gly Gly Ala Gly Ile Pro Asn Asn Glu Glu
               165                 170                 175
   ggt tgg aat gcg gta aaa gaa tac gct gat cca att gta gaa atg tta       1275
   Gly Trp Asn Ala Val Lys Glu Tyr Ala Asp Pro Ile Val Glu Met Leu
           180                 185                 190
   cgt gat agc ggg aac gca gat gac aat att atc att gtg ggt agt cca       1323
   Arg Asp Ser Gly Asn Ala Asp Asp Asn Ile Ile Ile Val Gly Ser Pro
       195                 200                 205
   aac tgg agt cag cgt cct gac tta gca gct gat aat cca att gat gat     1371
   Asn Trp Ser Gln Arg Pro Asp Leu Ala Ala Asp Asn Pro Ile Asp Asp
   210                 215                 220                 225
   cac cat aca atg tat act gtt cac ttc tac act ggt tca cat gct gct     1419
   His His Thr Met Tyr Thr Val His Phe Tyr Thr Gly Ser His Ala Ala
                   230                 235                 240
   tca act gaa agc tat ccg cct gaa act cct aac tct gaa aga gga aac     1467
   Ser Thr Glu Ser Tyr Pro Pro Glu Thr Pro Asn Ser Glu Arg Gly Asn
               245                 250                 255
   gta atg agt aac act cgt tat gcg tta gaa aac gga gta gca gta ttt     1515
   Val Met Ser Asn Thr Arg Tyr Ala Leu Glu Asn Gly Val Ala Val Phe
           260                 265                 270
   gca aca gag tgg gga act agc caa gca aat gga gat ggt ggt cct tac     1563
   Ala Thr Glu Trp Gly Thr Ser Gln Ala Asn Gly Asp Gly Gly Pro Tyr
       275                 280                 285
   ttt gat gaa gca gat gta tgg att gag ttt tta aat gaa aac aac att     1611
   Phe Asp Glu Ala Asp Val Trp Ile Glu Phe Leu Asn Glu Asn Asn Ile
   290                 295                 300                 305
   agc tgg gct aac tgg tct tta acg aat aaa aat gaa gta tct ggt gca     1659
   Ser Trp Ala Asn Trp Ser Leu Thr Asn Lys Asn Glu Val Ser Gly Ala
                   310                 315                 320
   ttt aca cca ttc gag tta ggt aag tct aac gca aca agt ctt gac cca     1707
   Phe Thr Pro Phe Glu Leu Gly Lys Ser Asn Ala Thr Ser Leu Asp Pro
               325                 330                 335
   ggg cca gac caa gta tgg gta cca gaa gag tta agt ctt tct gga gaa     1755
   Gly Pro Asp Gln Val Trp Val Pro Glu Glu Leu Ser Leu Ser Gly Glu
           340                 345                 350
   tat gta cgt gct cgt att aaa ggt gtg aac tat gag cca atc gac cgt     1803
   Tyr Val Arg Ala Arg Ile Lys Gly Val Asn Tyr Glu Pro Ile Asp Arg
       355                 360                 365
   aca aaa tac acg aaa gta ctt tgg gac ttt aat gat gga acg aag caa     1851
   Thr Lys Tyr Thr Lys Val Leu Trp Asp Phe Asn Asp Gly Thr Lys Gln
   370                 375                 380                 385
   gga ttt gga gtg aat gga gat tct cca gtt gaa gat gta gtt att gag     1899
   Gly Phe Gly Val Asn Gly Asp Ser Pro Val Glu Asp Val Val Ile Glu
                   390                 395                 400
   aat gaa gcg ggc gct tta aaa ctt tca gga tta gat gca agt aat gat     1947
   Asn Glu Ala Gly Ala Leu Lys Leu Ser Gly Leu Asp Ala Ser Asn Asp
               405                 410                 415
   gtt tct gaa ggt aat tac tgg gct aat gct cgt ctt tct gcc gac ggt     1995
   Val Ser Glu Gly Asn Tyr Trp Ala Asn Ala Arg Leu Ser Ala Asp Gly
           420                 425                 430
   tgg gga aaa agt gtt gat att tta ggt gct gaa aaa ctt act atg gat     2043
   Trp Gly Lys Ser Val Asp Ile Leu Gly Ala Glu Lys Leu Thr Met Asp
       435                 440                 445
   gtg att gtt gat gag ccg acc acg gta tca att gct gca att cca caa     2091
   Val Ile Val Asp Glu Pro Thr Thr Val Ser Ile Ala Ala Ile Pro Gln
   450                 455                 460                 465
   ggg cca tca gcc aat tgg gtt aat cca aat cgt gca att aag gtt gag     2139
   Gly Pro Ser Ala Asn Trp Val Asn Pro Asn Arg Ala Ile Lys Val Glu
                   470                 475                 480
   cca act aat ttc gta ccg tta gga gat aag ttt aaa gcg gaa tta act     2187
   Pro Thr Asn Phe Val Pro Leu Gly Asp Lys Phe Lys Ala Glu Leu Thr
               485                 490                 495
   ata act tca gct gac tct cca tcg tta gaa gct att gcg atg cat gct 2235
   Ile Thr Ser Ala Asp Ser Pro Ser Leu Glu Ala Ile Ala Met His Ala
   500 505 510
   gaa aat aac aac atc aac aac atc att ctt ttt gta gga act gaa ggt 2283
   Glu Asn Asn Asn Ile Asn Asn Ile Ile Leu Phe Val Gly Thr Glu Gly
   515 520 525
   gct gat gtt atc tat tta gat aac att aaa gta att gga aca gaa gtt 2331
   Ala Asp Val Ile Tyr Leu Asp Asn Ile Lys Val Ile Gly Thr Glu Val
   530 535 540 545
   gaa att cca gtt gtt cat gat cca aaa gga gaa gct gtt ctt cct tct 2379
   Glu Ile Pro Val Val His Asp Pro Lys Gly Glu Ala Val Leu Pro Ser
   550 555 560
   gtt ttt gaa gac ggt aca cgt caa ggt tgg gac tgg gct gga gag tct 2427
   Val Phe Glu Asp Gly Thr Arg Gln Gly Trp Asp Trp Ala Gly Glu Ser
   565 570 575
   ggt gtg aaa aca gct tta aca att gaa gaa gca aac ggt tct aac gcg 2475
   Gly Val Lys Thr Ala Leu Thr Ile Glu Glu Ala Asn Gly Ser Asn Ala
   580 585 590
   tta tca tgg gaa ttt gga tac cca gaa gta aaa cct agt gat aac tgg 2523
   Leu Ser Trp Glu Phe Gly Tyr Pro Glu Val Lys Pro Ser Asp Asn Trp
   595 600 605
   gca aca gct cca cgt tta gat ttc tgg aaa tct gac ttg gtt cgc ggt 2571
   Ala Thr Ala Pro Arg Leu Asp Phe Trp Lys Ser Asp Leu Val Arg Gly
   610 615 620 625
   gaa aat gat tat gta act ttt gat ttc tat cta gat cca gtt cgt gca 2619
   Glu Asn Asp Tyr Val Thr Phe Asp Phe Tyr Leu Asp Pro Val Arg Ala
   630 635 640
   aca gaa ggc gca atg aat atc aat tta gta ttc cag cca cct act aac 2667
   Thr Glu Gly Ala Met Asn Ile Asn Leu Val Phe Gln Pro Pro Thr Asn
   645 650 655
   ggg tat tgg gta caa gca cca aaa acg tat acg att aac ttt gat gaa 2715
   Gly Tyr Trp Val Gln Ala Pro Lys Thr Tyr Thr Ile Asn Phe Asp Glu
   660 665 670
   tta gag gaa gcg aat caa gta aat ggt tta tat cac tat gaa gtg aaa 2763
   Leu Glu Glu Ala Asn Gln Val Asn Gly Leu Tyr His Tyr Glu Val Lys
   675 680 685
   att aac gta aga gat att aca aac att caa gat gac acg tta cta cgt 2811
   Ile Asn Val Arg Asp Ile Thr Asn Ile Gln Asp Asp Thr Leu Leu Arg
   690 695 700 705
   aac atg atg atc att ttt gca gat gta gaa agt gac ttt gca ggg aga 2859
   Asn Met Met Ile Ile Phe Ala Asp Val Glu Ser Asp Phe Ala Gly Arg
   710 715 720
   gtc ttt gta gat aat gtt cgt ttt gag ggg gct gct act act gag ccg 2907
   Val Phe Val Asp Asn Val Arg Phe Glu Gly Ala Ala Thr Thr Glu Pro
   725 730 735
   gtt gaa cca gag cca gtt gat cct ggc gaa gag acg ccg cct gtc gat 2955
   Val Glu Pro Glu Pro Val Asp Pro Gly Glu Glu Thr Pro Pro Val Asp
   740 745 750
   gag aag gaa gcg aaa aaa gaa caa aaa gaa gca gag aaa gaa gag aaa 3003
   Glu Lys Glu Ala Lys Lys Glu Gln Lys Glu Ala Glu Lys Glu Glu Lys
   755 760 765
   gaa gca gta aaa gaa gaa aag aaa gaa gct aaa gaa gaa aag aaa gca 3051
   Glu Ala Val Lys Glu Glu Lys Lys Glu Ala Lys Glu Glu Lys Lys Ala
   770 775 780 785
   atc aaa aat gag gct acg aaa aaa taatctaata aactagttat agggttatct 3105
   Ile Lys Asn Glu Ala Thr Lys Lys
   790
   aaaggtctga tgcagatctt ttagataacc tttttttgca taactggaca tagaatggtt 3165
   attaaagaaa gcaaggtgtt tatacgatat taaaaaggta gcgattttaa attgaaacct 3225
   ttaataatgt cttgtgatag aatgatgaag taatttaaga gggggaaacg aagtgaaaac 3285
   ggaaatttct agtagaagaa aaacagacca agaaatactg caagctt 3332
<210> 4
   <211> 822
   <212> PRT
   <213> Bacillus sp. KSM-64
<400> 4
   Met Met Leu Arg Lys Lys Thr Lys Gln Leu Ile Ser Ser Ile Leu Ile
   -25 -20 -15
   Leu Val Leu Leu Leu Ser Leu Phe Pro Thr Ala Leu Ala Ala Glu Gly
   -10 -5 -1 1
   Asn Thr Arg Glu Asp Asn Phe Lys His Leu Leu Gly Asn Asp Asn Val
   5 10 15
   Lys Arg Pro Ser Glu Ala Gly Ala Leu Gln Leu Gln Glu Val Asp Gly
   20 25 30 35
   Gln Met Thr Leu Val Asp Gln His Gly Glu Lys Ile Gln Leu Arg Gly
   40 45 50
   Met Ser Thr His Gly Leu Gln Trp Phe Pro Glu Ile Leu Asn Asp Asn
   55 60 65
   Ala Tyr Lys Ala Leu Ala Asn Asp Trp Glu Ser Asn Met Ile Arg Leu
   70 75 80
   Ala Met Tyr Val Gly Glu Asn Gly Tyr Ala Ser Asn Pro Glu Leu Ile
   85 90 95
   Lys Ser Arg Val Ile Lys Gly Ile Asp Leu Ala Ile Glu Asn Asp Met
   100 105 110 115
   Tyr Val Ile Val Asp Trp His Val His Ala Pro Gly Asp Pro Arg Asp
   120 125 130
   Pro Val Tyr Ala Gly Ala Glu Asp Phe Phe Arg Asp Ile Ala Ala Leu
   135 140 145
   Tyr Pro Asn Asn Pro His Ile Ile Tyr Glu Leu Ala Asn Glu Pro Ser
   150 155 160
   Ser Asn Asn Asn Gly Gly Ala Gly Ile Pro Asn Asn Glu Glu Gly Trp
   165 170 175
   Asn Ala Val Lys Glu Tyr Ala Asp Pro Ile Val Glu Met Leu Arg Asp
   180 185 190 195
   Ser Gly Asn Ala Asp Asp Asn Ile Ile Ile Val Gly Ser Pro Asn Trp
   200 205 210
   Ser Gln Arg Pro Asp Leu Ala Ala Asp Asn Pro Ile Asp Asp His His
   215 220 225
   Thr Met Tyr Thr Val His Phe Tyr Thr Gly Ser His Ala Ala Ser Thr
   230 235 240
   Glu Ser Tyr Pro Pro Glu Thr Pro Asn Ser Glu Arg Gly Asn Val Met
   245 250 255
   Ser Asn Thr Arg Tyr Ala Leu Glu Asn Gly Val Ala Val Phe Ala Thr
   260 265 270 275
   Glu Trp Gly Thr Ser Gln Ala Asn Gly Asp Gly Gly Pro Tyr Phe Asp
   280 285 290
   Glu Ala Asp Val Trp Ile Glu Phe Leu Asn Glu Asn Asn Ile Ser Trp
   295 300 305
   Ala Asn Trp Ser Leu Thr Asn Lys Asn Glu Val Ser Gly Ala Phe Thr
   310 315 320
   Pro Phe Glu Leu Gly Lys Ser Asn Ala Thr Ser Leu Asp Pro Gly Pro
   325 330 335
   Asp Gln Val Trp Val Pro Glu Glu Leu Ser Leu Ser Gly Glu Tyr Val
   340 345 350 355
   Arg Ala Arg Ile Lys Gly Val Asn Tyr Glu Pro Ile Asp Arg Thr Lys
   360 365 370
   Tyr Thr Lys Val Leu Trp Asp Phe Asn Asp Gly Thr Lys Gln Gly Phe
   375 380 385
   Gly Val Asn Gly Asp Ser Pro Val Glu Asp Val Val Ile Glu Asn Glu
   390 395 400
   Ala Gly Ala Leu Lys Leu Ser Gly Leu Asp Ala Ser Asn Asp Val Ser
   405 410 415
   Glu Gly Asn Tyr Trp Ala Asn Ala Arg Leu Ser Ala Asp Gly Trp Gly
   420 425 430 435
   Lys Ser Val Asp Ile Leu Gly Ala Glu Lys Leu Thr Met Asp Val Ile
   440 445 450
   Val Asp Glu Pro Thr Thr Val Ser Ile Ala Ala Ile Pro Gln Gly Pro
   455 460 465
   Ser Ala Asn Trp Val Asn Pro Asn Arg Ala Ile Lys Val Glu Pro Thr
   470 475 480
   Asn Phe Val Pro Leu Gly Asp Lys Phe Lys Ala Glu Leu Thr Ile Thr
   485 490 495
   Ser Ala Asp Ser Pro Ser Leu Glu Ala Ile Ala Met His Ala Glu Asn
   500 505 510 515
   Asn Asn Ile Asn Asn Ile Ile Leu Phe Val Gly Thr Glu Gly Ala Asp
   520 525 530
   Val Ile Tyr Leu Asp Asn Ile Lys Val Ile Gly Thr Glu Val Glu Ile
   535 540 545
   Pro Val Val His Asp Pro Lys Gly Glu Ala Val Leu Pro Ser Val Phe
   550 555 560
   Glu Asp Gly Thr Arg Gln Gly Trp Asp Trp Ala Gly Glu Ser Gly Val
   565 570 575
   Lys Thr Ala Leu Thr Ile Glu Glu Ala Asn Gly Ser Asn Ala Leu Ser
   580 585 590 595
   Trp Glu Phe Gly Tyr Pro Glu Val Lys Pro Ser Asp Asn Trp Ala Thr
   600 605 610
   Ala Pro Arg Leu Asp Phe Trp Lys Ser Asp Leu Val Arg Gly Glu Asn
   615 620 625
   Asp Tyr Val Thr Phe Asp Phe Tyr Leu Asp Pro Val Arg Ala Thr Glu
   630 635 640
   Gly Ala Met Asn Ile Asn Leu Val Phe Gln Pro Pro Thr Asn Gly Tyr
   645 650 655
   Trp Val Gln Ala Pro Lys Thr Tyr Thr Ile Asn Phe Asp Glu Leu Glu
   660 665 670 675
   Glu Ala Asn Gln Val Asn Gly Leu Tyr His Tyr Glu Val Lys Ile Asn
   680 685 690
   Val Arg Asp Ile Thr Asn Ile Gln Asp Asp Thr Leu Leu Arg Asn Met
   695 700 705
   Met Ile Ile Phe Ala Asp Val Glu Ser Asp Phe Ala Gly Arg Val Phe
   710 715 720
   Val Asp Asn Val Arg Phe Glu Gly Ala Ala Thr Thr Glu Pro Val Glu
   725 730 735
   Pro Glu Pro Val Asp Pro Gly Glu Glu Thr Pro Pro Val Asp Glu Lys
   740 745 750 755
   Glu Ala Lys Lys Glu Gln Lys Glu Ala Glu Lys Glu Glu Lys Glu Ala
   760 765 770
   Val Lys Glu Glu Lys Lys Glu Ala Lys Glu Glu Lys Lys Ala Ile Lys
   775 780 785
   Asn Glu Ala Thr Lys Lys
   790
<210> 5
   <211> 2343
   <212> DNA
   <213> Bacillus sp.pHSP-K38
<220>
   <221> CDS
   <222> (580).. (2067)
   <223>
<220>
   <221> sig_peptide
   <222> (580).. (627)
   <223>
<220>
   <221> mat_peptide
   <222> (628).. ()
   <223>
<400> 5
   agatctagca ggatttgccg atgcaaccgg cttatattta gagggaattt ctttttaaat     60
   tgaatacgga ataaaatcag gtaaacaggt cctgatttta tttttttgaa tttttttgag     120
   aactaaagat tgaaatagaa gtagaagaca acggacataa gaaaattgta ttagttttaa    180
   ttatagaaaa cgcttttcta taattattta tacctagaac gaaaatactg tttcgaaagc     240
   ggtttactat aaaaccttat attccggctc tttttttaaa cagggggtga aaattcactc     300
   tagtattcta atttcaacat gctataataa atttgtaaga cgcaatatac atcttttttt    360
   tatgatattt gtaagcggtt aaccttgtgc tatatgccga tttaggaagg gggtagattg    420
   agtcaagtag tcataattta gataacttat aagttgttga gaagcaggag agaatctggg    480
   ttactcacaa gttttttaaa acattatcga aagcactttc ggttatgctt atgaatttag     540
   ctatttgatt caattacttt aataatttta ggaggtaat atg atg tta aga aag       594
                                              Met Met Leu Arg Lys
                                                      -15
   aaa aca aag cag ttg ggt cga cca gca caa gcc gat gga ttg aac ggt      642
   Lys Thr Lys Gln Leu Gly Arg Pro Ala Gln Ala Asp Gly Leu Asn Gly
       -10                 -5                  -1  1               5
   acg atg atg cag tat tat gag tgg cat ttg gaa aac gac ggg cag cat      690
   Thr Met Met Gln Tyr Tyr Glu Trp His Leu Glu Asn Asp Gly Gln His
                   10                  15                  20
   tgg aat cgg ttg cac gat gat gcc gca gct ttg agt gat gct ggt att      738
   Trp Asn Arg Leu His Asp Asp Ala Ala Ala Leu Ser Asp Ala Gly Ile
               25                  30                  35
   aca gct att tgg att ccg cca gcc tac aaa ggt aat agt cag gcg gat      786
   Thr Ala Ile Trp Ile Pro Pro Ala Tyr Lys Gly Asn Ser Gln Ala Asp
           40                  45                  50
   gtt ggg tac ggt gca tac gat ctt tat gat tta gga gag ttc aat caa      834
   Val Gly Tyr Gly Ala Tyr Asp Leu Tyr Asp Leu Gly Glu Phe Asn Gln
       55                  60                  65
   aag ggt act gtt cga acg aaa tac gga act aag gca cag ctt gaa cga      882
   Lys Gly Thr Val Arg Thr Lys Tyr Gly Thr Lys Ala Gln Leu Glu Arg
   70                  75                  80                  85
   gct att ggg tcc ctt aaa tct aat gat atc aat gta tac gga gat gtc      930
   Ala Ile Gly Ser Leu Lys Ser Asn Asp Ile Asn Val Tyr Gly Asp Val
                   90                  95                  100
   gtg atg aat cat aaa atg gga gct gat ttt acg gag gca gtg caa gct      978
   Val Met Asn His Lys Met Gly Ala Asp Phe Thr Glu Ala Val Gln Ala
               105                 110                 115
   gtt caa gta aat cca acg aat cgt tgg cag gat att tca ggt gcc tac     1026
   Val Gln Val Asn Pro Thr Asn Arg Trp Gln Asp Ile Ser Gly Ala Tyr
           120                 125                 130
   acg att gat gcg tgg acg ggt ttc gac ttt tca ggg cgt aac aac gcc     1074
   Thr Ile Asp Ala Trp Thr Gly Phe Asp Phe Ser Gly Arg Asn Asn Ala
       135                 140                 145
   tat tca gat ttt aag tgg aga tgg ttc cat ttt aat ggt gtt gac tgg     1122
   Tyr Ser Asp Phe Lys Trp Arg Trp Phe His Phe Asn Gly Val Asp Trp
   150                 155                 160                 165
   gat cag cgc tat caa gaa aat cat att ttc cgc ttt gca aat acg aac     1170
   Asp Gln Arg Tyr Gln Glu Asn His Ile Phe Arg Phe Ala Asn Thr Asn
                   170                 175                 180
   tgg aac tgg cga gtg gat gaa gag aac ggt aat tat gat tac ctg tta     1218
   Trp Asn Trp Arg Val Asp Glu Glu Asn Gly Asn Tyr Asp Tyr Leu Leu
               185                 190                 195
   gga tcg aat atc gac ttt agt cat cca gaa gta caa gat gag ttg aag     1266
   Gly Ser Asn Ile Asp Phe Ser His Pro Glu Val Gln Asp Glu Leu Lys
           200                 205                 210
   gat tgg ggt agc tgg ttt acc gat gag tta gat ttg gat ggt tat cgt     1314
   Asp Trp Gly Ser Trp Phe Thr Asp Glu Leu Asp Leu Asp Gly Tyr Arg
       215                 220                 225
   tta gat gct att aaa cat att cca ttc tgg tat aca tct gat tgg gtt     1362
   Leu Asp Ala Ile Lys His Ile Pro Phe Trp Tyr Thr Ser Asp Trp Val
   230                 235                 240                 245
   cgg cat cag cgc aac gaa gca gat caa gat tta ttt gtc gta ggg gaa     1410
   Arg His Gln Arg Asn Glu Ala Asp Gln Asp Leu Phe Val Val Gly Glu
                   250                 255                 260
   tat tgg aag gat gac gta ggt gct ctc gaa ttt tat tta gat gaa atg     1458
   Tyr Trp Lys Asp Asp Val Gly Ala Leu Glu Phe Tyr Leu Asp Glu Met
               265                 270                 275
   aat tgg gag atg tct cta ttc gat gtt cca ctt aat tat aat ttt tac     1506
   Asn Trp Glu Met Ser Leu Phe Asp Val Pro Leu Asn Tyr Asn Phe Tyr
           280                 285                 290
   cgg gct tca caa caa ggt gga agc tat gat atg cgt aat att tta cga     1554
   Arg Ala Ser Gln Gln Gly Gly Ser Tyr Asp Met Arg Asn Ile Leu Arg
       295                 300                 305
   gga tct tta gta gaa gcg cat ccg atg cat gca gtt acg ttt gtt gat     1602
   Gly Ser Leu Val Glu Ala His Pro Met His Ala Val Thr Phe Val Asp
   310                 315                 320                 325
   aat cat gat act cag cca ggg gag tca tta gag tca tgg gtt gct gat     1650
   Asn His Asp Thr Gln Pro Gly Glu Ser Leu Glu Ser Trp Val Ala Asp
                   330                 335                 340
   tgg ttt aag cca ctt gct tat gcg aca att ttg acg cgt gaa ggt ggt     1698
   Trp Phe Lys Pro Leu Ala Tyr Ala Thr Ile Leu Thr Arg Glu Gly Gly
               345                 350                 355
   tat cca aat gta ttt tac ggt gat tac tat ggg att cct aac gat aac     1746
   Tyr Pro Asn Val Phe Tyr Gly Asp Tyr Tyr Gly Ile Pro Asn Asp Asn
           360                 365                 370
   att tca gct aaa aaa gat atg att gat gag ctg ctt gat gca cgt caa    1794
   Ile Ser Ala Lys Lys Asp Met Ile Asp Glu Leu Leu Asp Ala Arg Gln
       375                 380                 385
   aat tac gca tat ggc acg cag cat gac tat ttt gat cat tgg gat gtt    1842
   Asn Tyr Ala Tyr Gly Thr Gln His Asp Tyr Phe Asp His Trp Asp Val
   390                 395                 400                 405
   gta gga tgg act agg gaa gga tct tcc tcc aga cct aat tca ggc ctt    1890
   Val Gly Trp Thr Arg Glu Gly Ser Ser Ser Arg Pro Asn Ser Gly Leu
                   410                 415                 420
   gcg act att atg tcg aat gga cct ggt ggt tcc aag tgg atg tat gta    1938
   Ala Thr Ile Met Ser Asn Gly Pro Gly Gly Ser Lys Trp Met Tyr Val
               425                 430                 435
   gga cgt cag aat gca gga caa aca tgg aca gat tta act ggt aat aac    1986
   Gly Arg Gln Asn Ala Gly Gln Thr Trp Thr Asp Leu Thr Gly Asn Asn
           440                 445                 450
   gga gcg tcc gtt aca att aat ggc gat gga tgg ggc gaa ttc ttt acg    2034
   Gly Ala Ser Val Thr Ile Asn Gly Asp Gly Trp Gly Glu Phe Phe Thr
       455                 460                 465
   aat gga gga tct gta tcc gtg tac gtg aac caa taacaaaaag ccttgagaag  2087
   Asn Gly Gly Ser Val Ser Val Tyr Val Asn Gln
   470                 475                 480
   ggattcctcc ctaactcaag gctttcttta tgtcgcttag ctttacgctt ctacgacttt  2147
   gaagcttggg gatccgtcga gacaaggtaa aggataaaac agcacaattc caagaaaaac  2207
   acgatttaga acctaaaaag aacgaatttg aactaactca taaccgagag gtaaaaaaag  2267
   aacgaagtcg agatcaggga atgagtttat aaaataaaaa aagcacctga aaaggtgtct   2327
   ttttttgatg tctaga                                                   2343
<210> 6
   <211> 496
   <212> PRT
   <213> Bacillus sp.pHSP-K38
<400> 6
   Met Met Leu Arg Lys Lys Thr Lys Gln Leu Gly Arg Pro Ala Gln Ala
   -15 -10 -5 -1
   Asp Gly Leu Asn Gly Thr Met Met Gln Tyr Tyr Glu Trp His Leu Glu
   1 5 10 15
   Asn Asp Gly Gln His Trp Asn Arg Leu His Asp Asp Ala Ala Ala Leu
   20 25 30
   Ser Asp Ala Gly Ile Thr Ala Ile Trp Ile Pro Pro Ala Tyr Lys Gly
   35 40 45
   Asn Ser Gln Ala Asp Val Gly Tyr Gly Ala Tyr Asp Leu Tyr Asp Leu
   50 55 60
   Gly Glu Phe Asn Gln Lys Gly Thr Val Arg Thr Lys Tyr Gly Thr Lys
   65 70 75 80
   Ala Gln Leu Glu Arg Ala Ile Gly Ser Leu Lys Ser Asn Asp Ile Asn
   85 90 95
   Val Tyr Gly Asp Val Val Met Asn His Lys Met Gly Ala Asp Phe Thr
   100 105 110
   Glu Ala Val Gln Ala Val Gln Val Asn Pro Thr Asn Arg Trp Gln Asp
   115 120 125
   Ile Ser Gly Ala Tyr Thr Ile Asp Ala Trp Thr Gly Phe Asp Phe Ser
   130 135 140
   Gly Arg Asn Asn Ala Tyr Ser Asp Phe Lys Trp Arg Trp Phe His Phe
   145 150 155 160
   Asn Gly Val Asp Trp Asp Gln Arg Tyr Gln Glu Asn His Ile Phe Arg
   165 170 175
   Phe Ala Asn Thr Asn Trp Asn Trp Arg Val Asp Glu Glu Asn Gly Asn
   180 185 190
   Tyr Asp Tyr Leu Leu Gly Ser Asn Ile Asp Phe Ser His Pro Glu Val
   195 200 205
   Gln Asp Glu Leu Lys Asp Trp Gly Ser Trp Phe Thr Asp Glu Leu Asp
   210 215 220
   Leu Asp Gly Tyr Arg Leu Asp Ala Ile Lys His Ile Pro Phe Trp Tyr
   225 230 235 240
   Thr Ser Asp Trp Val Arg His Gln Arg Asn Glu Ala Asp Gln Asp Leu
   245 250 255
   Phe Val Val Gly Glu Tyr Trp Lys Asp Asp Val Gly Ala Leu Glu Phe
   260 265 270
   Tyr Leu Asp Glu Met Asn Trp Glu Met Ser Leu Phe Asp Val Pro Leu
   275 280 285
   Asn Tyr Asn Phe Tyr Arg Ala Ser Gln Gln Gly Gly Ser Tyr Asp Met
   290 295 300
   Arg Asn Ile Leu Arg Gly Ser Leu Val Glu Ala His Pro Met His Ala
   305 310 315 320
   Val Thr Phe Val Asp Asn His Asp Thr Gln Pro Gly Glu Ser Leu Glu
   325 330 335
   Ser Trp Val Ala Asp Trp Phe Lys Pro Leu Ala Tyr Ala Thr Ile Leu
   340 345 350
   Thr Arg Glu Gly Gly Tyr Pro Asn Val Phe Tyr Gly Asp Tyr Tyr Gly
   355 360 365
   Ile Pro Asn Asp Asn Ile Ser Ala Lys Lys Asp Met Ile Asp Glu Leu
   370 375 380
   Leu Asp Ala Arg Gln Asn Tyr Ala Tyr Gly Thr Gln His Asp Tyr Phe
   385 390 395 400
   Asp His Trp Asp Val Val Gly Trp Thr Arg Glu Gly Ser Ser Ser Arg
   405 410 415
   Pro Asn Ser Gly Leu Ala Thr Ile Met Ser Asn Gly Pro Gly Gly Ser
   420 425 430
   Lys Trp Met Tyr Val Gly Arg Gln Asn Ala Gly Gln Thr Trp Thr Asp
   435 440 445
   Leu Thr Gly Asn Asn Gly Ala Ser Val Thr Ile Asn Gly Asp Gly Trp
   450 455 460
   Gly Glu Phe Phe Thr Asn Gly Gly Ser Val Ser Val Tyr Val Asn Gln
   465 470 475 480
<210> 7
   <211> 22
   <212> DNA
   <213> Artificial Sequence
<400> 7
   aaggatgata atccgtcccg tg 22
<210> 8
   <211> 38
   <212> DNA
   <213> Artificial Sequence
<400> 8
   gttatccgct cacaattcgg atggtcatca atcactag 38
<210> 9
   <211> 38
   <212> DNA
   <213> Artificial Sequence
<400> 9
   cgtcgtgact gggaaaactg cgaaatcaga cggtgtac 38
<210> 10
   <211> 20
   <212> DNA
   <213> Artificial Sequence
<400> 10
   cgtegeetat cggcgggcac 20
<210> 11
   <211> 25
   <212> DNA
   <213> Artificial Sequence
<400> 11
   atgtatatag gaggttggtg gtatg 25
<210> 12
   <211> 38
   <212> DNA
   <213> Artificial Sequence
<400> 12
   gttatccgct cacaattcgc tctgacatgt caacctcc 38
<210> 13
   <211> 38
   <212> DNA
   <213> Artificial Sequence
<400> 13
   cgtcgtgact gggaaaacag atgagaaagg aggagaag 38
<210> 14
   <211> 23
   <212> DNA
   <213> Artificial Sequence
<400> 14
   ataactgtta ctatataatg gcc 23
<210> 15
   <211> 20
   <212> DNA
   <213> Artificial Sequence
<400> 15
   gctggggatg acgaatccga 20
<210> 16
   <211> 38
   <212> DNA
   <213> Artificial Sequence
<400> 16
   gttatccgct cacaattctc accttcatta tggaccac 38
<210> 17
   <211> 38
   <212> DNA
   <213> Artificial Sequence
<400> 17
   cgtcgtgact gggaaaacca ccgtctcgaca aattccg 38
<210> 18
   <211> 20
   <212> DNA
   <213> Artificial Sequence
<400> 18
   gttgccaagc gcgatatagg 20
<210> 19
   <211> 25
   <212> DNA
   <213> Artificial Sequence
<400> 19
   tatacaggga ttatcagtat tgagc 25
<210> 20
   <211> 38
   <212> DNA
   <213> Artificial Sequence
<400> 20
   gttatccgct cacaattctt ttctccttgt tggatctg 38
<210> 21
   <211> 38
   <212> DNA
   <213> Artificial Sequence
<400> 21
   cgtcgtgact gggaaaacgg ggataacgat ttatgaag 38
<210> 22
   <211> 30
   <212> DNA
   <213> Artificial Sequence
<400> 22
   ttttgtaata atgatatgaa gctagtgttg 30
<210> 23
   <211> 20
   <212> DNA
   <213> Artificial Sequence
<400> 23
   atatccagcc ctgcctcttc 20
<210> 24
   <211> 58
   <212> DNA
   <213> Artificial Sequence
<400> 24
   ctgtgtgaaa ttgttatccg ctcacaattc gaaatttcct cctaaagcga tcataacg 58
<210> 25
   <211> 51
   <212> DNA
   <213> Artificial Sequence
<400> 25
   gtcgttttac aacgtcgttg actgggaaaa cccacaagct gctaacgtta c 51
<210> 26
   <211> 20
   <212> DNA
   <213> Artificial Sequence
<400> 26
   tcctgtttgg gctcctgttg 20
<210> 27
   <211> 26
   <212> DNA
   <213> Artificial Sequence
<400> 27
   tgtttatgta tggcggcctg cgggac 26
<210> 28
   <211> 38
   <212> DNA
   <213> Artificial Sequence
<400> 28
   gttatccgct cacaattcag ctttccatat atctcacc 38
<210> 29
   <211> 38
   <212> DNA
   <213> Artificial Sequence
<400> 29
   cgtcgtgact gggaaaacac ggtctgctga tgactgac 38
<210> 30
   <211> 20
   <212> DNA
   <213> Artificial Sequence
<400> 30
   gcgtttactt aagatgtcga 20
<210> 31
   <211> 39

   <212> DNA <213> Artificial Sequence
<400> 31 tttctagcgt ttcggcaaat tgagttaag 39
<210> 32
   <211> 38
   <212> DNA
   <213> Artificial Sequence
<400> 32
   gttatccgct cacaattcct tactttcata cggctcac 38
<210> 33
   <211> 38
   <212> DNA
   <213> Artificial Sequence
<400> 33
   cgtcgtgact gggaaaacga gacgtggcgc tcaccaac 38
<210> 34
   <211> 29
   <212> DNA
   <213> Artificial Sequence
<400> 34
   cggattaaaa aaagaatatc gcggacagc 29
<210> 35
   <211> 20
   <212> DNA
   <213> Artificial Sequence
<400> 35
   tgccgctgcc cgccggagag 20
<210> 36
   <211> 38
   <212> DNA
   <213> Artificial Sequence
<400> 36
   gttatccgct cacaattcaa ggtgtagaac ttccgttg 38
<210> 37 <211> 38 <212> DNA <213> Artificial Sequence <400> 37 cgtcgtgact gggaaaacac catcaacagc ccctacac 38
<210> 38 <211> 24 <212> DNA <213> Artificial Sequence <400> 38 tcaaataaag gcggcattca gtcc 24
<210> 39 <211> 22 <212> DNA <213> Artificial Sequence <400> 39 ataatggtat ccaaatccac gc 22
<210> 40 <211> 38 <212> DNA <213> Artificial Sequence <400> 40 gttatccgct cacaattcat tcagtcatat gtatcacc 38
<210> 41 <211> 38 <212> DNA <213> Artificial Sequence <400> 41 cgtcgtgact gggaaaacga tccatcatac acagcatg 38
<210> 42 <211> 28 <212> DNA <213> Artificial Sequence <400> 42 cacttctcaa cggaggggat ttcacatc 28
<210> 43 <211> 20 <212> DNA <213> Artificial Sequence <400> 43 taatggagga gagaaggccg 20
<210> 44 <211> 38 <212> DNA <213> Artificial Sequence <400> 44 gttatccgct cacaattcag tcgcccatga agcatgag 38
<210> 45
   <211> 42
   <212> DNA
   <213> Artificial Sequence
<400> 45
   cgtcgtgact gggaaaacac caaaaaatgc tgagctgaca gc 42
<210> 46
   <211> 26
   <212> DNA
   <213> Artificial Sequence
<400> 46
   ttgccaatga tgaggaaaaa ggaacc 26
<210> 47
   <211> 26
   <212> DNA
   <213> Artificial Sequence
<400> 47
   ctgaacgtcttgaataaaaaagcagg 26
<210> 48
   <211> 38
   <212> DNA
   <213> Artificial Sequence
<400> 48
   gttatccgct cacaattcgc tgaagtttca tatccatc 38
<210> 49
   <211> 38
   <212> DNA
   <213> Artificial Sequence
<400> 49
   cgtcgtgact gggaaaacat tccgtcatcg gcagcgag 38
<210> 50
   <211> 20
   <212> DNA
   <213> Artificial Sequence
<400> 50
   agcggtttac aagttggagg 20
<210> 51
   <211> 22
   <212> DNA
   <213> Artificial Sequence
<400> 51
   atttcagaag gcatacttca ag 22
<210> 52
   <211> 38
   <212> DNA
   <213> Artificial Sequence
<400> 52
   gttatccgct cacaattcca tacttggtgt tgtcatcg 38
<210> 53
   <211> 40
   <212> DNA
   <213> Artificial Sequence
<400> 53
   cgtcgtgact gggaaaacca taatcagtaa aaaggcggtc 40
<210> 54
   <211> 20
   <212> DNA
   <213> Artificial Sequence
<400> 54
   ttctgaccgc tctggcaacc 20
<210> 55
   <211> 20
   <212> DNA
   <213> Artificial Sequence
<400> 55
   ataatgcccg cttcccaacc 20
<210> 56
   <211> 38
   <212> DNA
   <213> Artificial Sequence
<400> 56
   gttatccgct cacaattccg atcctcagct cctttgtc 38
<210> 57
   <211> 38
   <212> DNA
   <213> Artificial Sequence
<400> 57
   cgtcgtgact gggaaaactc atctgatacc gattaacc 38
<210> 58
   <211> 20
   <212> DNA
   <213> Artificial Sequence
<400> 58
   caactgaatc cgaaggaatg 20
<210> 59
   <211> 20
   <212> DNA
   <213> Artificial Sequence
<400> 59
   tcggggtcat gccgagcggt 20
<210> 60
   <211> 38
   <212> DNA
   <213> Artificial Sequence
<400> 60
   gttatccgct cacaattcca atgttgccat tttcatcc 38
<210> 61
<211> 38
   <212> DNA
   <213> Artificial Sequence
<400> 61
   cgtcgtgact gggaaaactt gtacgagaat caacgctg 38
<210> 62
   <211> 20
   <212> DNA
   <213> Artificial Sequence
<400> 62
   cacggcaatg cattcttcgg 20
<210> 63
   <211> 21
   <212> DNA
   <213> Artificial Sequence
<400> 63
   agatctgtcg gccaggttta c 20
<210> 64
   <211> 38
   <212> DNA
   <213> Artificial Sequence
<400> 64
   gttatccgct cacaattctg atttttctgt catgtctc 61
<210> 65
   <211> 38
   <212> DNA
   <213> Artificial Sequence
<400> 65
   cgtcgtgact gggaaaacgg tagagatgtg caccgaaa 38
<210> 66
   <211> 20
   <212> DNA
   <213> Artificial Sequence
<400> 66
   gagtcagacg gcatcgatga 20
<210> 67
   <211> 23
   <212> DNA
   <213> Artificial Sequence
<400> 67
   ttctgattca ttttcactgc tgg 23
<210> 68
   <211> 38
   <212> DNA
   <213> Artificial Sequence
<400> 68
   gttatccgct cacaattcaa cggataattc ttccaatc 38
<210> 69
   <211> 37 <212> DNA
   <213> Artificial Sequence
<400> 69
   cgtcgtgact gggaaaactg tccatgaagt caaatcc 37
<210> 70
   <211> 20
   <212> DNA
   <213> Artificial Sequence
<400> 70
   cgctgaaata ttctctcgca 20
<210> 71
   <211> 21
   <212> DNA
   <213> Artificial Sequence
<400> 71
   cgccgctttc accgcggatt c 21
<210> 72
   <211> 38
   <212> DNA
   <213> Artificial Sequence
<400> 72
   gttatccgct cacaattcct ttgaccactg tatgaacc 38
<210> 73
   <211> 38

   <212> DNA
   <213> Artificial Sequence
<400> 73
   cgtcgtgact gggaaaacac tcgtctaacg aataatcc 38
<210> 74
   <211> 20
   <212> DNA
   <213> Artificial Sequence
<400> 74
   tgtcatcacg gaatttgacg 20
<210> 75
   <211> 30

   <212> DNA <213> Artificial Sequence
<400> 75
   ccaaattatc ctttgtgagc gcggaatcag 30
<210> 76
   <211> 38
   <212> DNA
   <213> Artificial Sequence
<400> 76
   gttatccgct cacaattccg tagatcgtaa tattgctc 38
<210> 77
   <211> 38
   <212> DNA
   <213> Artificial Sequence
<400> 77
   cgtcgtgact gggaaaacag cttagaaagt caaccaag 38
<210> 78
   <211> 20
   <212> DNA
   <213> Artificial Sequence
<400> 78
   tttgagcatc agcacaagcc 20
<210> 79
   <211> 21
   <212> DNA
   <213> Artificial Sequence
<400> 79
   tgtagcagaa gcagtcgaat t 21
<210> 80
   <211> 40
   <212> DNA
   <213> Artificial Sequence
<400> 80
   ctaatgggtg ctttagttga caattacgca gctgtcatgt 40
<210> 81
   <211> 41
   <212> DNA
   <213> Artificial Sequence
<400> 81
   ctgccccgtt agttgaagaa ctgataaacc gtgaaaaagt g 41
<210> 82
   <211> 20
   <212> DNA
   <213> Artificial Sequence
<400> 82
   cctttgaaaa aggctcccgt 20
<210> 83
   <211> 29
   <212> DNA
   <213> Artificial Sequence
<400> 83
   gttttccaag tctgccgata aaaatatgc 29
<210> 84
   <211> 38

   <212> DNA <213> Artificial Sequence
<400> 84
   gttatccgct cacaattcat gcttcatgta cctacacc 38
<210> 85
   <211> 38
   <212> DNA
   <213> Artificial Sequence
<400> 85
   cgtcgtgact gggaaaacca attaacgatt cgcatacc 38
<210> 86
   <211> 30
   <212> DNA
   <213> Artificial Sequence
<400> 86
   aaaaagaaga agtcacagta cagaacgtgg 30
<210> 87
   <211> 23
   <212> DNA
   <213> Artificial Sequence
<400> 87
   atttttcgcc atcttgaatt ttc 23
<210> 88
   <211> 40
   <212> DNA
   <213> Artificial Sequence
<400> 88
   ctaatgggtg ctttagttgg atgatcctct cgttgaactg 40
<210> 89
   <211> 39
   <212> DNA
   <213> Artificial Sequence
<400> 89
   ctgccccgtt agttgaaggg atgagccttc agaaaagtt 39
<210> 90
   <211> 20
   <212> DNA
   <213> Artificial Sequence
<400> 90
   gccggacaga gatctgtatg 20
<210> 91
   <211> 45
   <212> DNA
   <213> Artificial Sequence
<400> 91
   gaagaaggtt tttatgttga cgcttttttg cccaatactg tataa 45
<210> 92
   <211> 45
   <212> DNA
   <213> Artificial Sequence
<400> 92
   caaaaaagcg tcaacataaa aaccttcttc aactaacggg gcagg 45
<210> 93
   <211> 30
   <212> DNA
   <213> Artificial Sequence
<400> 93
   aagacgagta cttttctctc taaatcactt 30
<210> 94
   <211> 30
   <212> DNA
   <213> Artificial Sequence
<400> 94
   aactcgatca aatggtgaca ggacagcatc 30
<210> 95
   <211> 45
   <212> DNA
   <213> Artificial Sequence
<400> 95
   ggagaataaa gaccctcttc aactaaagca cccattagtt caaca 45
<210> 96
   <211> 45
   <212> DNA
   <213> Artificial Sequence
<400> 96
   tgctttagtt gaagagggtc tttattctcc cacagggttt cgttt 45
<210> 97
   <211> 45
   <212> DNA
   <213> Artificial Sequence
<400> 97
   tttttatatt acagcgagtt ggcgttaaat gaatgaagcg ataga 45
<210> 98

   <211> 45 <212> DNA
   <213> Artificial Sequence
<400> 98
   atttaacgcc aactcgctgt aatataaaaa ccttcttcaa ctaac 45
<210> 99
   <211> 30
   <212> DNA
   <213> Artificial Sequence
<400> 99
   ttgattgatg ataaattcag gcaggtgcag 30
<210> 100
   <211> 30
   <212> DNA
   <213> Artificial Sequence
<400> 100
   caaagcttga gaaatgttcc catgctcttg 30
<210> 101

   <211> 45
   <212> DNA
   <213> Artificial Sequence
<400> 101 caggaggaac atatctcttc aactaaagca cccattagtt caaca 45
<210> 102
   <211> 45
   <212> DNA
   <213> Artificial Sequence
<400> 102

   tgctttagtt gaagagatat gttcctcctg ttccgggctg ccccg 45
<210> 103
   <211> 25
   <212> DNA
   <213> Artificial Sequence
<400> 103
   attccagtta ctcgtaatat agttg 25
<210> 104
   <211> 38
   <212> DNA
   <213> Artificial Sequence
<400> 104
   gttatccgct cacaattcac ttcatcatcc attagctc 38
<210> 105
   <211> 38
   <212> DNA
   <213> Artificial Sequence
<400> 105
   cgtcgtgact gggaaaacct gctccaaatc cgatttcc 38
<210> 106
   <211> 23
   <212> DNA
   <213> Artificial Sequence
<400> 106

   gtcctgcatt tttcgaagtc tgg 23
<210> 107
   <211> 30

   <212> DNA <213> Artificial Sequence
<400> 107
   tacacatcct tcaaacaagt ctgaacaaac 30
<210> 108
   <211> 45
   <212> DNA
   <213> Artificial Sequence
<400> 108
   tgctttagtt gaagattacc agttccataa ttccacctcg ccgac 45
<210> 109
   <211> 45
   <212> DNA
   <213> Artificial Sequence
<400> 109
   tttttatatt acagcgtgtg tataccattg tatctgtaga tacga 45
<210> 110
   <211> 30
   <212> DNA
   <213> Artificial Sequence
<400> 110
   gctatgatca ttgtaacgaa aggaaagggg 30
<210> 111
   <211> 45
   <212> DNA
   <213> Artificial Sequence
<400> 111
   ttatggaact ggtaatcttc aactaaagca cccattagtt caaca 45
<210> 112
   <211> 45
   <212> DNA
   <213> Artificial Sequence
<400> 112
   caatggtata cacacgctgt aatataaaaa ccttcttcaa ctaac 45
<210> 113
   <211> 30
   <212> DNA
   <213> Artificial Sequence
<400> 113
   aatctgaaca agaaaaagga gctgctcctc 30
<210> 114
   <211> 45
   <212> DNA
   <213> Artificial Sequence
<400> 114
   tgctttagtt gaagaattca atctccctcc atgtcagctt attta 45
<210> 115
   <211> 45
   <212> DNA
   <213> Artificial Sequence
<400> 115
   tttttatatt acagcagaaa cgcctgaaat gaaccggccc tatag 45
<210> 116
   <211> 30
   <212> DNA
   <213> Artificial Sequence
<400> 116
   tgtttgacaa aggtagaacg tctgcttatc 30
<210> 117
   <211> 45
   <212> DNA
   <213> Artificial Sequence
<400> 117
   ggagggagat tgaattcttc aactaaagca cccattagtt caaca 45
<210> 118
   <211> 45
   <212> DNA
   <213> Artificial Sequence
<400> 118
   atttcaggcg tttctgctgt aatataaaaa ccttcttcaa ctaac 45
<210> 119
   <211> 18
   <212> DNA
   <213> Artificial Sequence
<400> 119
   gaattgtgag cggataac 18
<210> 120
   <211> 18
   <212> DNA
   <213> Artificial Sequence
<400> 120
   gttttcccag tcacgacg 18
<210> 121
   <211> 19
   <212> DNA
   <213> Artificial Sequence
<400> 121
   caactaaagc acccattag 19
<210> 122
   <211> 18

   <212> DNA <213> Artificial Sequence
<400> 122
   cttcaactaa cggggcag 18